(19) 

Europäisches
Patentamt
European
Patent Office
Office européen
des brevets

(11) **EP 4 467 944 B1**

(12) **EUROPEAN PATENT SPECIFICATION**

(45) Date of publication and mention
of the grant of the patent:
**19.02.2025 Bulletin 2025/08**

(21) Application number: **23175204.9**

(22) Date of filing: **24.05.2023**

(51) International Patent Classification (IPC):
*G01J 3/44* *(2006.01)*     *G01N 21/359* *(2014.01)*
*G01N 21/65* *(2006.01)*     *G01N 21/35* *(2014.01)*
*G01N 21/39* *(2006.01)*     *G01J 3/12* *(2006.01)*
*G01J 3/28* *(2006.01)*     *G16C 20/20* *(2019.01)*

(52) Cooperative Patent Classification (CPC):
**G01N 21/65; G16C 20/20;** G01N 21/35;
G01N 2021/399; G01N 2201/121

(54) **SYSTEMS AND METHODS FOR IDENTIFYING SUBSTANCES CONTAINED IN A SAMPLE**

SYSTEME UND VERFAHREN ZUR IDENTIFIZIERUNG VON SUBSTANZEN IN EINER PROBE

SYSTÈMES ET PROCÉDÉS D'IDENTIFICATION DE SUBSTANCES CONTENUES DANS UN
ÉCHANTILLON

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**27.11.2024 Bulletin 2024/48**

(73) Proprietor: **Bruker Switzerland AG
8117 Fällanden (CH)**

(72) Inventors:
• **Fäh, Kevin
8604 Volketswil (CH)**

• **Prodan, Marc
8050 Zürich (CH)**

(74) Representative: **Bittner, Peter et al
Peter Bittner und Partner
Herrenwiesenweg 2
69207 Sandhausen (DE)**

(56) References cited:
CN-A- 112 834 451     CN-A- 113 176 225
US-A1- 2005 075 797     US-A1- 2009 210 194
US-A1- 2016 259 792     US-A1- 2021 364 441
US-A1- 2022 244 173     US-B1- 8 873 041

**Description**

**Technical Field**

The present invention generally relates to spectroscopic measurements of samples, and more particularly relates to identifying substances contained in a sample based on a measured spectrum obtained from the sample via an optical spectroscopy method.

**Background**

In optical spectroscopy, such as RAMAN, infrared (IR) and quantum-cascade laser (QCL), a measurement device with a spectrometer can acquire a spectrum of an unknown sample. This input spectrum can be regarded as a vector of N data pairs (x,y) where x represents the wavenumber and y the intensity. Given a measured RAMAN, IR or QCL input spectrum and a set of reference spectra of known pure substances from libraries, the task of component search or also named mixture analysis, is to identify the substance or multiple substances that are represented in the input spectrum of said sample. The task of component search is also referred to as "substance search" herein.

A necessary assumption for component (substance) search is that the substances (comprised by said sample) that contribute to the input spectrum, are represented in the reference libraries. Therefore, in order to increase the likelihood for substance identification, a naive measurement device typically uses a multitude of reference libraries. The fast and correct substance identification in these libraries represents a difficult challenge for the substance search algorithm.

Other challenges are the varying environmental and instrumental conditions under which the input spectrum is acquired compared to the reference spectra in the libraries. The variations typically lead to spectral distortions that need to be handled with correction techniques. It is therefore a challenge to handle these distortions in a compliant way to still being able to identify the correct substances.

Due to the linear nature of both, Lambert Beer's law in case of IR and QCL and inelastic scattering in case of RAMAN spectroscopy, regression techniques are a well working tool for substance search. When solving this task in a regression fashion, relative contributions of the components (substances) can be visually indicated by calculating a composite spectrum given the identified substances from the reference library.

The assumption is that the substances that are represented by the input spectrum are actually represented by respective reference spectra in the reference library. Therefore, it is typically beneficial to have as many reference spectra as possible being part of the analysis if the sample composition reflected by the input spectrum is totally unknown. On the other hand, this makes fast and qualitative analysis challenging, because it requires to deal with an overdetermined regression problem (more reference spectra K than data points N in the spectra).

Other challenges arise from the differences between a spectrum of the same substance measured under different measurement conditions (e.g., different device, different environment condition such as temperature, pressure etc.). Since a reference spectrum of a pure substance is in general acquired only once under specific measurement conditions, the substance search is confronted with possible spectral differences that may occur for an unknown substance measured under different conditions. Effects of these varying measurement conditions may affect the baseline, noise level, peak location and relative intensity of peaks in the spectra. Depending on the spectrum type (RAMAN, IR, QCL) certain effects are more significant and can affect the probability of finding the correct substances of the input spectrum.

Some prior art substance search solutions separate the correction of important spectral effects (such as for example the baseline, peak shift and intensity distortions) from the regression problem and apply the correction only once or in an iterative way. This can lead to suboptimal results. Typically, prior art solutions compute multicomponent results (e.g., $K$ components) by using the results of the $K$ - 1 component search. Such iterative multicomponent search is typically unsatisfactory, because it leads to a longer runtime until multicomponent search results are finally available. To reduce the runtime, such prior art approaches use only a limited number of the results $M$ of search stage $K$ - 1 for search stage $K$. This keeps the runtime linear in the number of reference spectra. However, due to the limiting number of results $M$, potential mixture components cannot be identified.

Due to the above-mentioned separation of spectral corrections from the regression problem and the iterative approach of computing the multicomponent (multi-substance) search results at stage $K$ from the results of stage $K$ - 1, errors are propagated and are difficult to correct.

US Patent Application US2016259792A1 discloses an approach for comparing sample spectrum of a sample substance to reference spectra to identify matches, thereby determining possibilities for what the sample substance is. Correction parameter(s) can be used for the sample spectrum and/or the reference spectrum. Initial value(s) for the correction parameter(s) can be applied to the sample spectrum and/or a reference spectrum, and a similarity score can be determined, wherein the system iteratively cycles through each correction parameter separately.

## Summary

Therefore, there is a need for systems and methods for fast and accurate analysis of substances contained in a sample using a general-purpose computer for time-constrained applications based on IR, QCL or Raman spectroscopy.

This is achieved by embodiments of the invention formulating a substance-search task with regression, filtering and corrections as one single optimization problem. For example, a convex optimization can be used which may be advantageous because a global optimum exists for any convex problem and advanced numerical solvers are widely available. Spectral correction tasks can be divided into baseline correction, peak shift correction and intensity correction. The usage of a correction or the degree of freedom for a correction can differ dependent on the type of the measured input spectrum (IR, RAMAN, QCL) and the measured or user selected spectral range on which the substance search should be performed. Spectral corrections of peak shift, intensity distortions and baseline can be handled best when respective matching reference spectra are available. On the other hand, matching reference spectra can be identified best when spectral corrections are applied in combination with the reference spectra. The spectral corrections and component filtering are combined into one comprehensive optimization problem that leads to a global optimum.

The herein disclosed approach uses a reference library which typically includes a large number of reference spectra associated with respective substances. For an optimized substance-search, all the reference spectra of the reference library are considered in a top-down approach for a successive reduction of the number of reference spectra until only reference spectra remain which reflect such substances contained in the sample. A reference library may include tens of thousands of reference spectra. The herein disclosed approach includes the creation of a hierarchical order of all reference spectra (HIT list) followed by batchwise component filtering. In both steps the above-mentioned comprehensive convex optimization may be used. In one embodiment, a computer-implemented method is provided for identifying one or more substances included in a sample based on a measured spectrum obtained from the sample via an optical spectroscopy method. The method may be executed by a general-purpose computer system which implements functional modules to perform the steps described in the following. The steps implement a substance search pipeline which allows the method to run also on a general-purpose computer with limited computing resources (e.g., 1 CPU core, 8GB RAM).

Initially, the system obtains the measured spectrum (input spectrum from sample with unknown composition) via an appropriate interface. Further, the system accesses the library comprising a plurality of reference spectra of known pure substances. Then, the substance search pipeline starts with a pre-processing phase (for the input spectrum and the reference spectra) followed by a single substance search phase (no combination of reference substances is considered, only single substance spectra) and a limited multi substance search phase (considering a limited set of reference spectra based on the result of the single component search) resulting in a substance identification result for said sample. In an optional embodiment, the limited multi-substance search phase is followed by an extended multi-substance search (considering all reference spectra in combination with each other to explain the input spectrum) which can further improve the accuracy of the substance identification result. The substance identification result includes a composition spectrum of found reference spectra, the relative contribution of the found reference components, the spectra corrections and evaluation metrics that explain the quality of the result in addition to the applied spectral correction.

The different phases are described in more detail in the following.

In the pre-processing phase, the measured spectrum and the plurality of reference spectra are pre-processed by applying a min-max normalization to each spectrum such that the intensity ranges of all spectra are within the interval [0, 1], and to ensure that all spectra match regarding the spectral range. Furthermore, all spectra are adjusted to have the same spectral range and spectral resolution, the latter being achieved by interpolation, for example.

Optionally, the pre-processing phase may further include the separation of the full spectral range into R analysis ranges $S_i$ $i \in [1, R]$. The analysis ranges can be used to apply intensity corrections. Advantageously, the analysis ranges can be chosen such that a split between them occurs at a local minimum between peaks or groups of peaks in the input spectrum. Further, additional spectrum type specific information, such as the information about the so-called "silent region" in RAMAN spectra, may be used for determining the analysis ranges. A peak position can be identified with a peak pick method. The region around a peak can be defined by the full width at half maximum of the peak. If peaks are close to each other (threshold) they can be grouped together.

After the pre-processing phase the single substance search phase is performed which includes:

a) solving an optimization problem with a first set of hyperparameters (e.g., the hyperparameter set H1 as defined in the detailed description) for all possible pairs of the measured spectrum with the reference spectra in the library. In general, a solver approximates the optimum via an iterative procedure. In case the solver fails to solve the problem, the problem may be solved again using another configuration. The optimization problem is formulated as a single optimization problem for regression, substance filtering and spectral corrections, wherein spectral corrections comprise corrections of peak shift and baseline, and in case of RAMAN spectroscopy, also comprise corrections of intensity distortion. The first set of hyperparameters is configured such that each reference spectrum is adapted to obtain the best possible matching result of each adapted reference spectrum with the measured spectrum. As in the single substance search phase the measured spectrum is only compared to single substance reference spectra, substance filtering is not needed and the respective hyperparameter for the filtering in the first set of hyperparameters is set to '0'.

b) determining a similarity score for each matching result using a similarity metric and ranking the reference spectra in accordance with the similarity scores of the respective adapted reference spectra.

The similarity metric scores the spectral match of two spectra. The metric is used to rank the results of the optimization problem by comparing the measured spectrum to the composite spectrum resulting from the optimization. The similarity metric may be a weighted average of a fit similarity metric and a peak similarity metric. The fit similarity metric is the coefficient of determination ($R^2$). The peak similarity metric is computed by splitting the measured spectrum into peak bands (with a peak pick method). For each peak band, the correlation between the measured spectrum and the composite spectrum in that peak band is computed. The peak similarity metric is the average over all band correlation values.

In the following, a general optimization problem is described which is appropriate for being used in accordance with the pipeline approach:

Given an input spectrum $y$ with $N$ measurement points we want to find a minimal number of pure reference spectra $z_i$ among the $K$ library spectra and their weight contribution $w_i$ that match the input spectrum. To compute an intensity correction the input spectrum and the library spectra may be split into R analysis ranges. In this case, for each number of reference spectra i $\in$ [1,$K$], there would be R individual weights $x_{i,j} \in$ [1,$R$], one per analysis range. A person skilled in the art can also formulate a general optimization problem when using only a single analysis range.

To correct peak shifts between the composite spectrum and the input spectrum, P peak correction polynomials are introduced, one per spectral range $C_i$ for i $\in$ [1,$P$]. The number of peak correction polynomials $P$ is defined by the number of significant peaks in the input spectrum (peaks with an intensity larger than a threshold). The width of the spectral range $C_i$ for a peak correction polynomial is defined by the width of the peak or peak group.

The indices notation $i,j$ is used to make it easier to differ between a specific library component $i$ (reference spectrum for substance i) and the corresponding analysis range $j$. The following constrained multi-objective optimization problem (OP) can be formulated:

$$argmin_x \, \beta \|Ax - y\|_2^2 + (1 - \beta)\|Ax - y\|_1 + \lambda \|x\|_2^2 +$$
$$\gamma \|x_{K,R}\|_1 +$$
$$\tau \|x_{BPoly3+}\|_1 +$$
$$\theta \sum_{i=1}^{P} x_{i_{PeakPolySum}} +$$
$$\mu \sum_{i=1}^{K} \sum_{j=1}^{R-1} \|x_{i,j+1} - x_{i,j}\|_2^2$$

where

$$x = \begin{pmatrix} x_{K,R} \\ x_{BPoly} \\ x_{PeakPoly_1} \\ ... \\ x_{PeakPoly_P} \end{pmatrix}$$

and $x_{1:P_{PeakPolySum}}$ are the optimization variables.

In this formulation the optimization variables x consist of:

- the component weights of the $K$ library spectra in the R analysis ranges $x_{i,j}$ i $\in$ [1, $K$], j $\in$ [1,$R$]
- the polynomial coefficients $x_{BPoly}$ of a Nth (e.g, N=7) order Legendre polynomial, approximating the baseline of the input mixture
- $x_{PeakPoly_1}$ up to $x_{PeakPoly_P}$ are the coefficients of the P peak correction polynomials, one per spectral range $C_i$ for $i \in$ [1, $P$]. Each peak correction polynomial is a N$^{th}$ order Legendre polynomial (N=7)

The optimization variables $x_{1:P_{PeakPolySum}}$ are the $P$ sums for each of the $P$ peak polynomials which approximate the integral of the polynomial in the spectral range $C_i$ for i E [1, P]

The columns of the Matrix $A \in \mathbb{R}^{N \times ((K*R)+BPoly+PeakPoly*P)}$,

$$A = [A_{K,R} \quad A_{BPoly} \quad A_{PeakPoly_1} \quad ... \quad A_{PeakPoly_P}]$$

are the $K$ standardized reference spectra, which are split into the $R$ analysis ranges $A_{K,R}$ (whereby the spectral range not covered by an analysis range is filled with zeros), the standardized Legendre polynomials for the baseline estimate $A_{BPoly}$, and the P standardized Legendre polynomials for the peak correction $A_{PeakPoly1}..A_{PeakPolyP}$. The standardization is computed with the formula $\dfrac{x-\mu}{\sigma}$, where $x$ is the polynomial or the reference spectrum, $\mu$ is the mean of the polynomial or the reference spectrum and $\sigma$ is the standard deviation of the polynomial or the reference spectrum.

The following constraints are imposed to the optimization problem:

i. The first constraint is

$$x_{K,R} \geq 0,$$

which means that the weights of the library spectra in each region are nonnegative.
ii. The second constraint is

$$A_{BPoly}x_{BPoly} \geq 0,$$

which demands the baseline approximation to be nonnegative.
iii. The third constraint is

$$A_{BPoly}x_{BPoly} \leq y,$$

which demands the baseline approximation to be smaller than the input spectrum. This constraint is only active when the reference spectra are not standardized which is the case in the "result computation" step of the component search explained later.
iv. There are two equality constraints per peak correction polynomial $A_{PeakPoly_i}x_{PeakPoly_i}$ $i \in [1,P]$, demanding the polynomial to be zero at the boundaries of the spectral range $C_i$

$$A_{PeakPoly_i}x_{PeakPoly_i} [\omega = C_{i_{start}}] = 0$$

$$A_{PeakPoly_i}x_{PeakPoly_i} [\omega = C_{i_{end}}] = 0$$

v. There are additionally two equality constraints per peak correction polynomial $A_{PeakPoly_i}x_{PeakPoly_i}$ $i \in [1,P]$, demanding the derivative of the polynomial to be zero at the boundaries of the spectral range $C_i$

$$\frac{dA_{PeakPoly_i}x_{PeakPoly_i}}{d\omega}[\omega = C_{i_{start}}] = 0$$

$$\frac{dA_{PeakPoly_i}x_{PeakPoly_i}}{d\omega}[\omega = C_{i_{end}}] = 0$$

vi. And one additional equality constraint per peak correction polynomial that makes $x_{iPeakPolySum}$ to approximate the integral of the peak correction polynomial via the sum

$$\sum_j A_{PeakPoly_i} \boldsymbol{x}_{PeakPoly_i}[j] = x_{i_{PeakPolySum}}$$

The optimization problem can be solved with different configurations of hyperparameters (sometimes also referred to as cost weight terms $[\beta, \lambda, \gamma, \tau, \theta, \mu]$) depending on the type of the input spectrum or the stage of the search method as described further down below. For example, the following cost terms may be used:

- The term $\beta \| Ax - \boldsymbol{y} \|_2^2 + (1 - \beta) \| Ax - \boldsymbol{y} \|_1$ is the regression term. This term makes sure that a composite spectrum matches the input spectrum, where the composite spectrum is the composition of the baseline, the weighted reference spectra and the peak shift and intensity corrections.

- Advantageously, the parameter $\beta$ is either 0 or 1. Which means that either the L2 norm or the L1 norm (cf., K. Nipp & D. Stoffer (2002) Lineare Algebra: Eine Einführung für Ingenieure unter besonderer Berücksichtigung numerischer Aspekte. (5. Aufl.) vdf Hochschulverlag AG) of the difference between the input mixture spectrum and the composite spectrum is minimized.

- A ridge term is added for numerical stability with weight $\lambda$ in $\lambda \| x \|_2^2$. A larger $\lambda$ implies a higher stability.

- To get a sparse solution w.r.t the components $\boldsymbol{x}_{K,R}$, this vector is minimized with the L1 norm and a cost weight of $\gamma$ in $\gamma \| \boldsymbol{x}_{K,R} \|_1$. This term serves as a filter to minimize the number of reference spectra that are used in the composite spectrum.

- The baseline polynomial terms larger than order 2 are also penalized with the L1 norm and cost weight $\tau$ in $\tau \| \boldsymbol{x}_{BPoly3+} \|_1$. This leads to a minimal support baseline to avoid overfitting.

- The integral of the $P$ peak shift polynomials approximated by the sum is minimized with weight $\theta$ in $\theta \sum_{i=1}^{P} x_{i_{PeakPolySum}}$. This is to not fit peaks but allow to compensate shifts. Therefore, the integral should be close to zero.

- To restrict the strength of the intensity correction and imply smoothness, the differences of the weights of adjacent regions $j$ and $j + 1$ for $j \in [1, R]$ for a component $i \in [1, K]$ are minimized by a L2 norm. This term is weighted by $\mu$ in the cost function in $\mu \sum_{i=1}^{K} \sum_{j=1}^{R-1} \| x_{i,j+1} - x_{i,j} \|_2^2$.

In case of using multiple analysis ranges for intensity corrections, all analysis ranges are taken into account when solving the single optimization problem.

The choice of the hyperparameters shifts the focus of the objective regarding the task of regression, filtering and correction. For example, setting $\gamma$ to zero eliminates the filtering effect of the L1 norm. As mentioned earlier, the first set of hyperparameters is configured such that each reference spectrum is adapted to obtain the best possible matching result of each adapted reference spectrum with the measured spectrum. In other words, the first set of hyperparameters (e.g., hyperparameter set H1 as disclosed in the detailed description) is configured to optimally adapt the reference spectra to the measured spectrum for a spectral comparison.

The result of the single substance search is a ranked list of reference spectra where substances associated with a reference spectrum having a high similarity with the measured input spectrum (high similarity score) are ranked higher than substances whose reference spectra having a low similarity score under the used similarity metrics. This intermediate result may already be provided to a user of the system.

After completion of the single substance search phase, a limited multi-substance search is performed by substance filtering (steps c, d), substance reduction and importance weighting (step e), and result calculation (steps f to h):

c) selecting a predefined number M of reference spectra from the ranking result starting with the reference spectrum having the highest similarity score and standardizing the selected reference spectra;
d) solving the optimization problem with a second set of hyperparameters (e.g., the hyperparameter set H2 as disclosed in the detailed description) with the measured spectrum and the M standardized reference spectra, wherein the second set of hyperparameters being configured such that the hyperparameter for substance filtering is emphasized to result in a set of mixture substance candidates. Advantageously, the size of the set of mixture substance candidates is at least one order of magnitude (i.e., at least a factor of 10) smaller than the number of selected reference spectra from the ranking result;

e) solving the optimization problem with a third set of hyperparameters (e.g., the hyperparameter set H3 as disclosed in the detailed description) with the measured spectrum and the standardized reference spectra associated with the potential mixture substances, wherein the third set of hyperparameters being configured such that the filtering effect is attenuated in relation to the second set of hyperparameters (i.e., $\gamma(\textit{third set}) < \gamma(\textit{second set})$) whereby substances are discarded until all remaining substances have a relative weight greater than a predefined threshold of significance, the relative weight being the normalized weight of the result of the optimization problem;

f) computing a plurality of composition batches from the normalized reference spectra associated with the remaining substances such that a composition batch is generated for each possible subset of the remaining substances which includes the remaining substance with the highest relative weight;

g) solving the optimization problem for each composition batch and the measured spectrum with the first set of hyperparameters (e.g., hyperparameter set H1) where filtering is eliminated ($\gamma = 0$) resulting in a composite spectrum per composition batch. Alternatively, a skilled person may use another appropriate set of hyperparameters as long as filtering is eliminated; and

h) evaluating the quality of each composite spectrum by evaluating the results of the optimization problem, as obtained by using the first set of hyperparameters, with said similarity metric scoring the spectral match of the respective composite spectrum with the measured spectrum, and providing the one or more substances associated with composite spectra having a quality above a predefined quality threshold as substances contained in the sample.

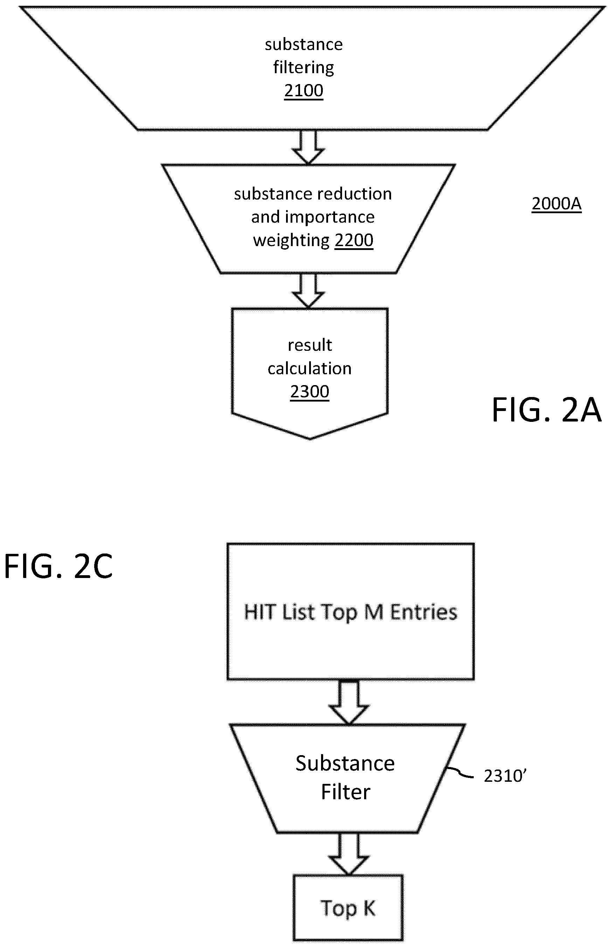

In the substance filtering steps c) and d), the selected predefined number M of standardized reference spectra from the ranking result with the highest similarity scores (top M entries of the HIT list) are used as input for solving the optimization problem with the second set of hyperparameters with the substance filter 2310' as illustrated in FIG. 2C. Thereby, the cost weights are chosen to strengthen the filtering effect. This filtering leads to a set of mixture substance candidates of size K«M. For example, in a well working setting, the second set of hyperparameters may be chosen such that K≈20 and M is in the range of [600,1000].

In the substance reduction and importance weighting step e), the predefined threshold of significance may be chosen in the order of 0.01 so that only substances with a relative weight larger than the threshold of significance are kept. The relative weight which is the normalized weight of the result of the optimization problem serves as an importance weight for a respective substance with regard to the importance of its contribution to a composite spectrum.

In the result calculation steps f) to h), the normalized reference spectra of the remaining weighted substances are used. In one implementation, the normalized reference spectra from the pre-processing step are used. Alternatively, the standardized spectra are de-standardized again resulting in the normalized reference spectra. Using the normalized reference spectra simplifies the interpretation of the relative contribution of a reference spectrum to the composite spectrum. A plurality of composition batches is then computed from the reference spectra associated with the remaining weighted substances such that a composition batch is generated for each combination of subsets of the K remaining substances which includes the remaining substance with the highest relative weight. In one implementation, this allows to compute a composite spectrum with the relative contribution of each substance from the set of K remaining substances with the associated K reference spectra. K composition batches are built from the remaining K substances, where each composition batch $Bc_i$ contains all combinations with i substances $i \in [1,K]$, where the substance with the highest weighting after the previous step e is part of each combination. That is, the creation of the K composition batches depends on the importance weight determined by the previous step.

For each composition batch, the optimization problem is solved with the first set of hyperparameters (or a similar set of hyperparameters where filtering is set to zero) resulting in a composite spectrum. An example, where no filtering is involved and the filtering term is eliminated (y=0), is given in the hyperparameter set H1. For each optimization problem result, again a weighted Fit and Peak Metric may be used as similarity metrics to evaluate the quality of the composite spectrum. That is, at this point this intermediate result may already be provided to a user of the system to indicate the substances which are contained in the measured sample. Thereby, the composite spectrum with the highest quality is associated with a high probability that the underlying combination of substances reflects the actual composition of the sample.

In an optional embodiment, the computer-implemented method can still be further improved by performing an extended multi-substance search after the limited multi-substance search phase. In an ideal situation the optimization problem would be solved with all available reference spectra at once. However, this is computationally too demanding for a standard general-purpose computer. Therefore, the herein proposed approach uses batchwise substance filtering with the goal to still consider as many reference spectra as possible together in one optimization problem but using the results of the single substance search and the limited multi-substance search to split the reference spectra of the library among batches. The batchwise substance filtering comprises the steps i to l.

i) composing a plurality of filter batches wherein each filter batch includes reference spectra associated with a top-ranked subset P of the set of potential mixture substances of step c), the reference spectra associated with the one or more substances provided by step h), and a predefined number of the remaining reference spectra from the library, and standardizing the reference spectra of each filter batch;

j) solving the optimization problem with the second set of hyperparameters for each filter batch;

k) consolidating the result sets of each filter batch by eliminating redundant substances from the consolidated result into a consolidated set of mixture substance candidates; and

l) reapplying steps d) to h) by using the reference spectra associated with the consolidated set of potential mixture substances instead of the selected reference spectra of step d).

In the batchwise substance filtering steps, the reference spectra are distributed among N filter batches. A set of substances, referred to as "all batch substances" is part of each batch. The "all batch substances" include the top $P \ll M$ entries of the HIT list of the single substance search and the substances from the results of the limited multi-substance search.

The remaining substances represented in the reference library which are not part of the "all batch substances" are distributed among the filter batches. The number of reference spectra per filter batch can be fixed and the resulting number of filter batches may depend on the number of selected reference spectra.

The optimization problem is solved per filter batch with the second set of hyperparameters emphasizing substance filtering. Advantageously, the optimization problems can be solved in parallel in case the computational resources support parallel processing.

From the results of each filter batch, the filtered components are consolidated.

The consolidated mixture substance candidates identified with the batchwise filtering step are then fed through the steps of substance filtering, substance reduction and importance weighting and the result calculation the same way as described in the limited multi-substance search. The computed results can then be provided to the user as further improved estimates for the composition of said sample. As the optional extended multi-substance search phase takes into account also reference spectra which were already filtered out in the preceding phases, the optional embodiment advantageously supports also the identification of substances which cause low intensity signals (e.g., trace elements in the sample that are of particular importance for forensics).

It is to be understood that both, the foregoing general description and the following detailed description are exemplary and explanatory only and are not restrictive of the invention as set out in the appended set of claims.

**Brief Description of the Drawings**

FIG. 1 is a block diagram of a computer system for identifying one or more substances contained in a sample based on a measured spectrum obtained from the sample via an optical spectroscopy according to an embodiment;

FIG. 2A illustrates a generic approach for substance identification in a sample;

FIG. 2B illustrates an example embodiment of a pipeline approach for a general-purpose computer to identify substances in a sample;

FIG. 2C illustrates details of a single substance search phase of the pipeline approach;

FIG. 3 illustrates details of an extended multi-substance search phase in an optional embodiment of the pipeline approach;

FIGs. 4A, 4B illustrate a simplified flow chart of a computer-implemented method for identifying one or more substances included in a sample based on a measured spectrum obtained from the sample via an optical spectroscopy method according to an embodiment;

FIGs 5A to 5C are example RAMAN spectra of two different substances and a mixture of such substances;

FIG. 6 illustrates the dividing of a measured spectrum into multiple analysis ranges according to an embodiment;

FIGs. 7A to 7C illustrate an example of peak shift correction in a composite spectrum;

FIGs. 8A to 8C illustrate an example of intensity correction in a composite spectrum; and

FIG. 9 is a diagram that shows an example of a generic computer device and a generic mobile computer device which may be used with the techniques described herein.

## Detailed Description

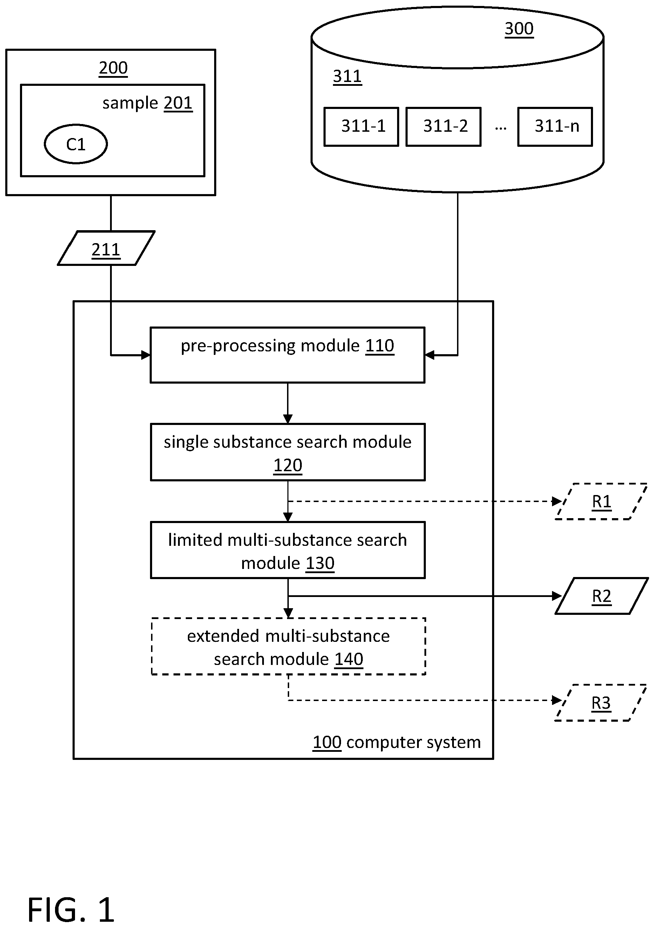

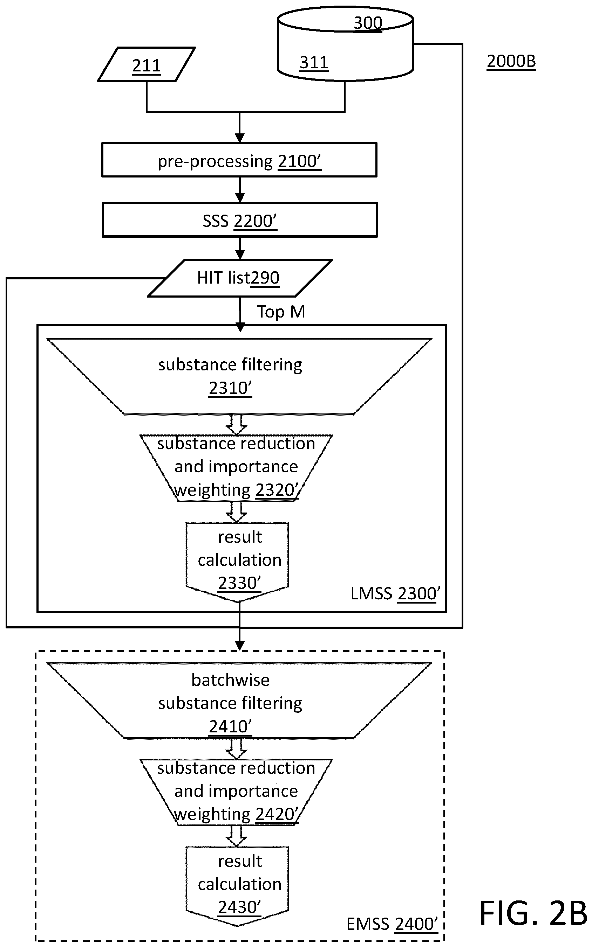

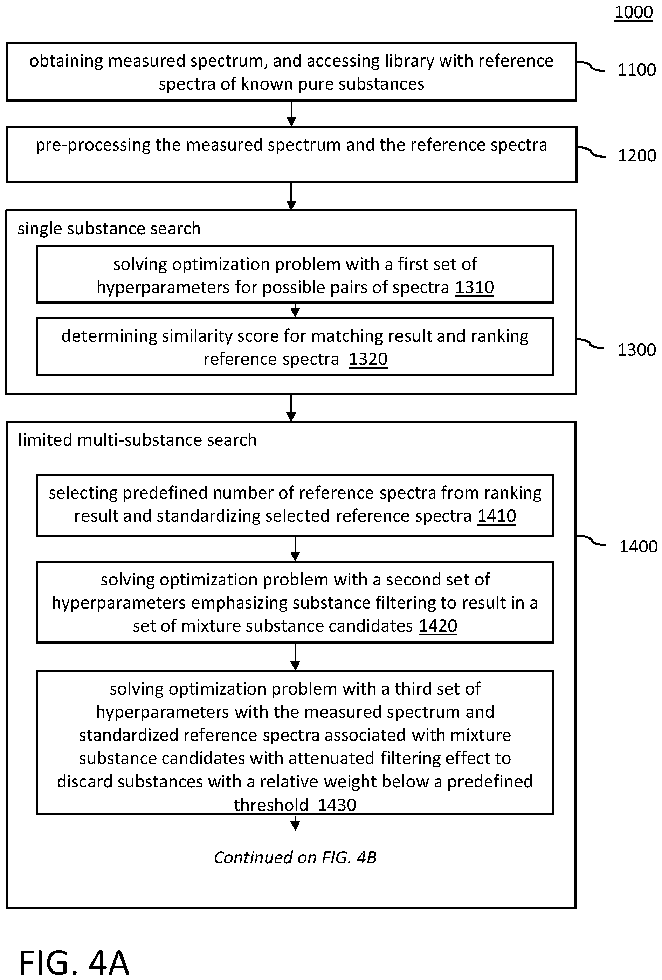

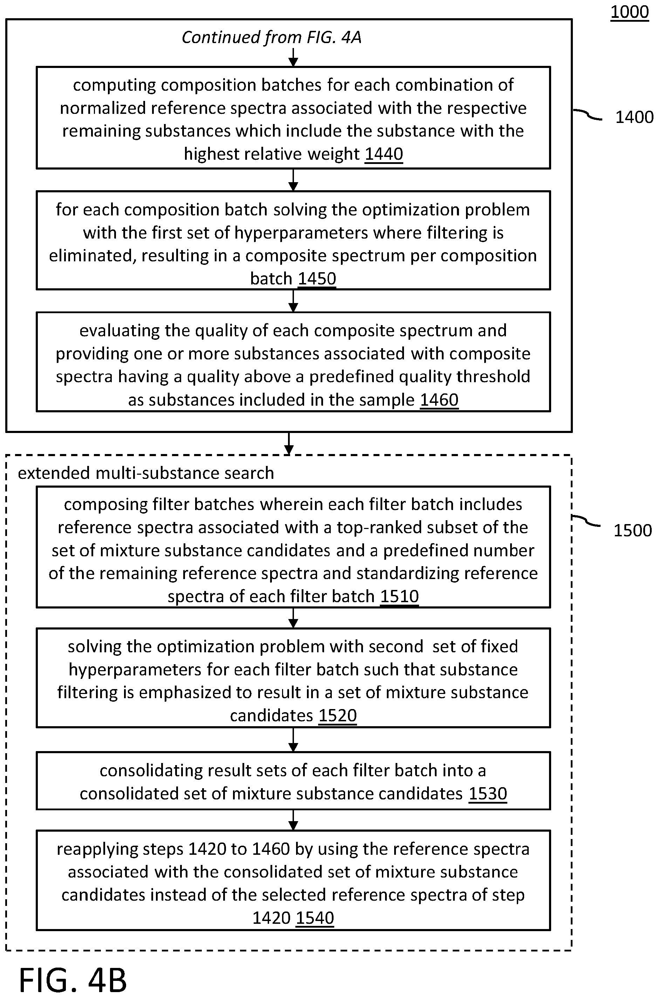

FIG. 1 is a block diagram of a computer system 100 for identifying one or more substances contained in a sample based on a measured spectrum 211 obtained from the sample via optical spectroscopy according to an embodiment. The embodiment reflects a pipeline approach 2000B for a general-purpose computer to identify substances in said sample as illustrated in more detail in FIG. 2B. Optional elements of the embodiment are illustrated by dashed lines. FIGs. 4A, 4B illustrate a simplified flow chart of a computer-implemented method 1000 for identifying one or more substances included in a sample based on a measured spectrum 211 obtained from the sample via an optical spectroscopy method according to an embodiment.

The system 100 will be described in the following in view of the computer-implemented method 1000 and the pipeline approach 2000B. The description will therefore refer to reference numbers used in such figures.

The computer system 100 is communicatively coupled with a data source 200 providing the measured spectrum 211 obtained from the sample via an optical spectroscopy method. The sample 201 contains an unknown composition C1 of one or more substances which is to be determined. The data source 200 may be a RAMAN, infrared (IR) or quantum-cascade laser (QCL) spectrometer which obtains the measured spectrum 211, or it may be a data storage which buffers the obtained spectrum. The system 100 is further communicatively coupled with at least one library 300 comprising a plurality 311 of reference spectra 311-1 to 311-n of known pure substances. In the following, the description refers to one library with reference spectra. However, a person skilled in the art can easily use reference spectra stored in multiple libraries. The reference spectra 311* have been measured using the same optical spectroscopy method as for the sample 201. Interfaces for exchanging data between spectrometers or data storage devices are well known in the art. Hence, the system 100 obtains 1100 the measured spectrum and accesses the library 300 via an appropriate interface.

The system has a pre-processing module 110 which is implemented by software and which is configured to pre-process 1200, 2100' the measured spectrum 211 and the plurality 311 of reference spectra by applying a min-max normalization to each spectrum such that the intensity ranges of all spectra are within the interval [0, 1]. Each spectrum can be regarded as a vector of $N$ data pairs $(x,y)$ where $x$ represents the wavenumber and $y$ represents the intensity at this wavenumber. Min-max normalization is a common way to normalize data. For every intensity value, the minimum value gets transformed into a 0, the maximum value gets transformed into a 1, and every other value gets transformed into a decimal between 0 and 1. Further, pre-processing module 110 ensures that all spectra have the same spectral range with the same spectral resolution. That is, in case the measured spectrum covers a different spectral range than the reference spectra, only overlapping ranges can be used. In case the wavenumbers of the intensity values are not identical, the spectra can be interpolated to derive the intensity values at the respective wavenumbers so that all spectra have the same spectral resolution. Typically, the reference spectra stored in a library have already identical spectral resolution. However, in case multiple libraries are used, there may be a need to adjust the spectral resolution of some reference spectra by using the above interpolation method.

Based on the pre-processed input/reference spectra, a search for the substances contained in the sample is performed. In an ideal world with unlimited computing resources, an ideal substance search 2000A would be possible as depicted in FIG. 2A. The ideal substance search 2000A would apply the optimization problem described above to all available reference spectra 311 at once. The result would be a set of substances with non-zero weights and correction terms for baseline, peak shift and intensity correction. From the set of substances with non-zero weights, different results with different number and combinations of components can be computed also by solving the presented optimization problem with a different task objective by changing the hyperparameters. The set of hyperparameters can be seen as a set of control parameters for solving the optimization problem. Theses control parameters can shift the emphasis between regression, filtering and corrections when solving the optimization problem. After solving the optimization problem with a specific set of hyperparameters $[\beta,\lambda,\gamma,\tau,\theta,\mu]$ in the substance filtering step 2100, mixture substance candidates can be identified. In the next step 2200, the number of substances is further reduced and weighted by importance. Depending on their importance, different combinations of substances can be chosen, and results can be computed for each combination of substances by solving the optimization problem with another set of hyperparameters that does not include filtering in the result calculation step 2300. The ideal substance search 2000A is an optimal result implementation. Since all reference spectra are considered at once, a true global optimum can be computed. But this setup would require advanced hardware capabilities due to the possibly large number of reference spectra in the libraries (typically in the order of tens of thousands). It is therefore not a suitable approach to be run on a commercial general-purpose computer as used in a typical spectrum analysis setup.

The details of the pipeline approach 2000B are described in the following. In the pipeline approach, a surrogate substance search pipeline solving the above-described optimization problem by splitting the task of solving the problem into multiple steps, substantially reduces the computational burden to a level which can be handled by a general-purpose computer. In a basic embodiment of the pipeline approach, the system uses a single substance search module 120 and a limited multi-substance search module 130. The result R2 provided by module 130 already provides a good estimate for the substances contained in the analyzed sample 201. The intermediate result R1 of the single substance search module may also be already communicated to a user of the system as an initial guess for the contained substances.

The single substance search module 120 performs 1300, 2200' the single substance search by:

- solving 1310 the OP with a first set of hyperparameters for all possible pairs of the measured spectrum 211 with the reference spectra 311 in the library 300. That is, the measured spectrum is always compared with one reference spectrum associated with a pure substance. Because the OP is formulated as a single optimization problem for regression, substance filtering and spectral corrections, different sets of hyperparameters can be used for solving the OP with different focusses in various steps of the pipeline approach. A focus on regression uses a set of hyperparameters which is different from a set of hyperparameters with a focus on filtering or spectral corrections. The first set of hyperparameters (e.g., H1) is configured to optimally adapt the reference spectra to the measured spectrum for a spectral comparison. Therefore, substance filtering is not required in the single substance search and the respective hyperparameter $\gamma$ is set to zero. For solving the OP, a standard solver for convex optimization problems can be used. Examples of solvers suitable for convex optimization problems are listed, for example, at: https://en.wikipedia.org/wiki/Convex_optimization.

In an example embodiment, three sets of hyperparameters {H1, H2, H3} for the parameters $[\beta,\lambda,\gamma,\tau,\theta,\mu]$ of the OP are used. In the example embodiment, the following parameters are the same for all three sets: $\beta = 1, \tau = 1, \theta = 10$. Setting $\beta$ to 1 means that only the squared loss is used as the cost for the regression task. The baseline polynomial coefficients for the degrees larger than 3 are penalized with the cost weight $\tau = 1$ and the integral of the peak correction polynomials are minimized with the cost weight $\theta = 10$.

The distinguishing parameters are:

- First set of hyperparameters H1: $\lambda = 10^{-6}, \gamma = 0, \mu = 0$ *or* 1

- Second set of hyperparameters H2: $\lambda = 10^{-4}, \gamma = 0.5, \mu = 0$

- Third set of hyperparameters H3: $\lambda = 10^{-4}, \gamma = 0, \mu = 0$

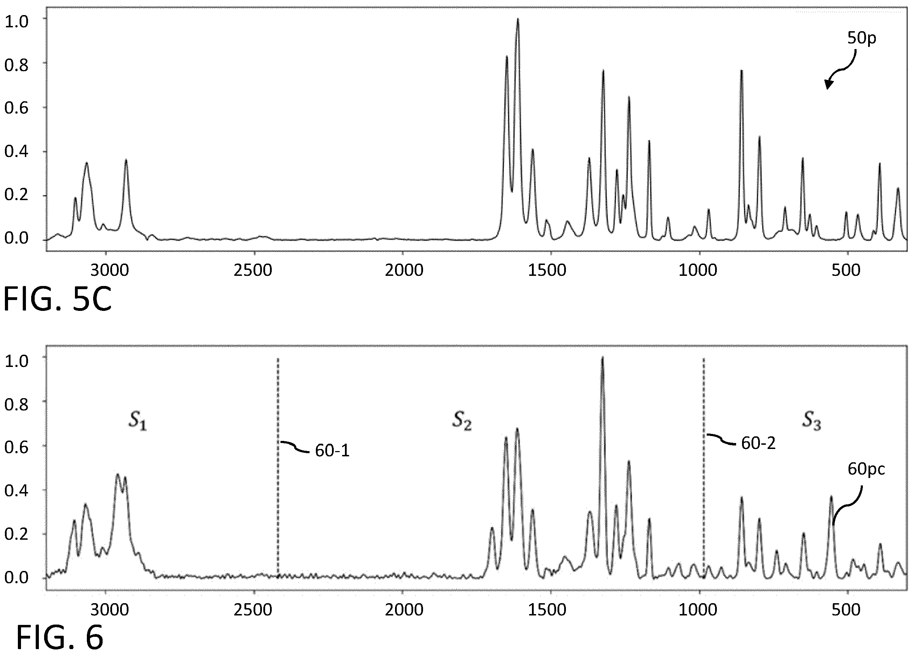

The cost weight term $\lambda$ for the L2-norm regularization of the component weights is smaller in set H1 because this set is only applied to very few reference spectra at once (advantageously maximum 10) and thus the need for numerical stabilization is minimal. The cost weight $\gamma$ for the L1-norm regularization of the component weights is non-zero if component filtering is applied as in the hyperparameter set H2. The cost weight term $\mu$ is used to penalize the variation in the component weights between neighboring analysis ranges. The term is set to one if an intensity correction is applied as in set H1. This is described in more detail in the context of FIG. 6.

These sets of hyperparameters were found during the development of the example embodiment by optimizing and manually tuning the parameters to achieve a stable and fast solver behavior and good results on a labeled dataset (measured spectra with known substance composition). The following description refers to the three sets of hyperparameters {H1, H2, H3}, although a person skilled in the art may use deviating sets of hyperparameters to achieve a corresponding functioning of the respective modules.

The result of the solving step 1310 provides adapted (single substance) reference spectra which are optimally adapted to the measured spectrum 211. The single substance search module 120 then determines 1320 a similarity score for each pair of measured spectrum and adapted reference spectrum using a similarity metric scoring the spectral match of two spectra. Advantageously, the above-described fit-and-peak metrics may be used. However, a person skilled in the art may also chose other appropriate similarity metrics for scoring the spectral match. The reference spectra are then ranked in accordance with the similarity scores of the respective adapted reference spectra in a HIT list 290. In other words, the HIT list 290 shows the reference spectra in descending order of the similarity score which is a first intermediate result R1 which can already give indications to a user with regard to respective substances which are most likely contained in the sample (substances associated with reference spectra having a high similarity score).

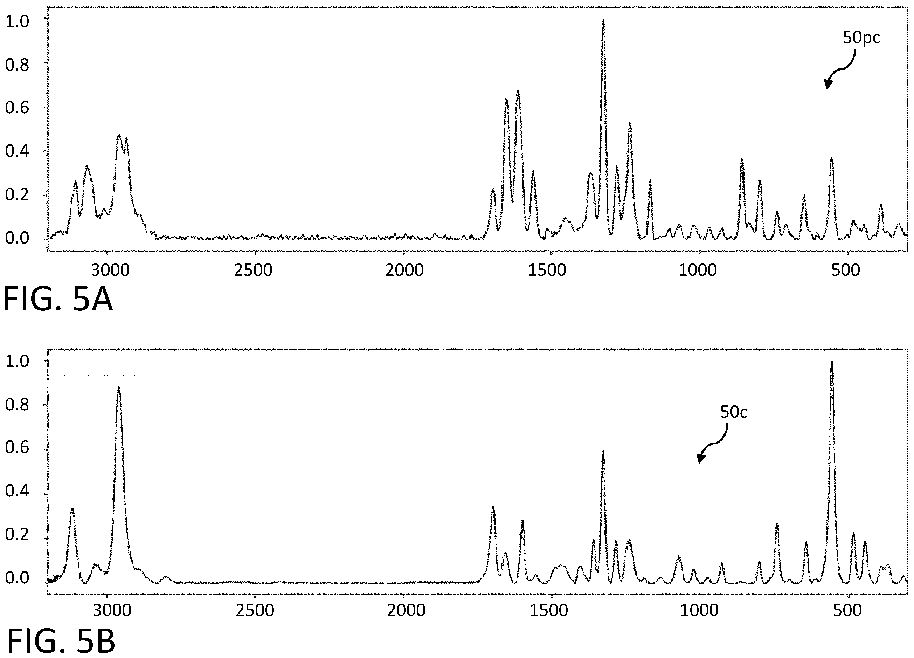

Turning briefly to FIGs. 5A to 5C, FIG. 5A shows an example of a measured RAMAN input spectrum 50pc of a sample containing a mixture of Paracetamol and Caffeine without any baseline distortions. FIGs. 5B and 5C are reference spectra from the library 300 wherein FIG. 5B is a RAMAN spectrum 50c of pure Caffeine and FIG. 5C is a RAMAN spectrum 50p for pure Paracetamol. The spectra already were pre-processed (y-axis: min-max normalized intensity range [0,1]) and cover the identical wavenumber range (x-axis). In the single substance search, module 120 would solve the optimization problem to adapt the spectra 50c and 50p such that they optimally match the measured spectrum 50pc. High similarity scores for the two pairs of spectra (50pc, 50c) and (50pc, 50p) indicate that both substances are likely contained in the sample.

However, to compute a more reliable mixture substance result, the limited multi-substance search module 130 performs 1400 a limited multi-substance search 2300" (LMSS) which also takes into account composite spectra generated from two or more different reference spectra. In a first sub-phase 2310' of the LMSS phase, module performs substance filtering using the HIT list from the SSS phase 2200'. For this purpose, module 130 selects 1410 a predefined number M of reference spectra from the HIT list 290 starting with the reference spectrum having the highest similarity score (i.e., the Top M entries of the HIT list). In other words, the LMSS phase is based on a limited subset of the reference spectra with the highest similarity scores. Advantageously, M is in the order of 500 to 2000 entries. As a consequence, the number of possible substance combinations is dramatically reduced (only combinations of the most similar reference spectra) compared to the previously discussed ideal approach where all possible combinations of all reference spectra would be considered. The selected reference spectra are standardized in accordance with the previously described standardization formula.

This allows to solve 1420 the optimization problem with a second set of hyperparameters (e.g., $H2$) with the measured spectrum 211 and all standardized reference spectra also on a general-purpose computer. The second set of hyperparameters is configured such that substance filtering is emphasized to result in a set of mixture substance candidates. In the example embodiment, for $H2$ $\gamma$ is set to 0.5. After this substance filtering 2310' phase, the resulting set of mixture substance candidates includes N potential mixture substances with N < M.

In the substance reduction and importance weighting phase 2320', module 130 solves 1430 the optimization problem with a third set of hyperparameters with the measured spectrum 211 and the N standardized reference spectra associated with the mixture substance candidates. Thereby, the third set of hyperparameters (e.g., H3) is configured such that the filtering effect is attenuated in relation to the second set of hyperparameters H2 (e.g., from $\gamma$ = 0.5 to $\gamma$ = 0 in the example embodiment) so that substances are discarded until all P remaining weighted substances have a relative weight (normalized weight of the result of the optimization problem) greater than a predefined threshold of significance (e.g., 0.01 relative weight). The candidate with highest relative weight is considered to be the most promising substance which is used as an anchor point for the following steps in the result calculation phase 2330'.

In phase 2330', an example embodiment of module 130 computes 1440 a plurality of composition batches from the normalized reference spectra (e.g., after de-standardizing the standardized spectra or by using the normalized spectra from the pre-processing phase) associated with the P remaining weighted substances. Each composition batch contains the candidate with the highest relative weight. The first series of composition batches includes all possible pairs of the remaining weighted substances. For example, if A is the substance with the highest relative weight, and if the remaining weighted substances are B, C, D, E then the first series of composition batches would include the combinations (A,B), (A,C), (A,D), (A,E). The order of the substances in the pairs plays no role for the generation of the composition batches. The second series of composition batches includes all possible doubles of the remaining weighted substances: (A,B,C), (A,B,D), (A,B,E), (A,C,D), (A,C,E), (A,D,E). The third series of composition batches includes all possible triples of the remaining weighted substances: (A,B,C,D), (A,B,C,E), (A,B,D,E), (A,C,D,E). The fourth series of composition batches includes all possible quadruples of all combinations of the remaining weighted substances. As there are only five weighted substances, there is only one batch in the third series: (A,B,C,D,E). In this embodiment, a composition batch is created for each combination of the weighted substance having the highest relative weight with any possible subset of the other remaining weighted substances.

Then, module 130 solves 1450 the optimization problem for each generated composition batch with the first set of hyperparameters resulting in a composite spectrum per composition batch. Alternatively, a further set of hyperparameters similar to the first set may be used as long as filtering is eliminated (i.e., $\gamma$ = 0). That is, in step 1450, the generated composite spectra are adapted to optimally fit to the measured spectrum 211. In a computing device supporting parallel processing, the optimization problems can be solved in parallel.

In step 1460, module 130 evaluates the quality of each composite spectrum by evaluating the results of the optimization problem (obtained by using the first set of hyperparameters) with said similarity metric. Further, the one or more substances associated with composite spectra having a quality above a predefined quality threshold are provided as set of substances R2 contained in the sample 201. The set R2 is already a very good estimate for the substances making up the composition C1 of sample 201.

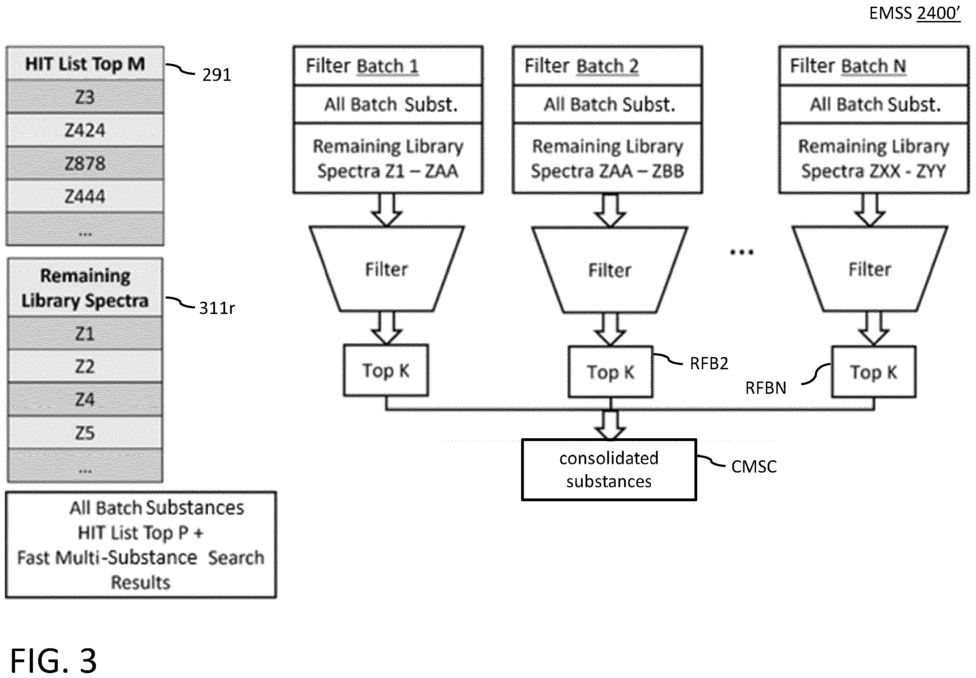

This estimate can be further improved by using the optional extended multi-substance search module 140. In this embodiment, an extended multi-substance search phase (EMSS) 1500, 2400' is performed by module 140 after the completion of the LMSS phase 1400, 2300'. The EMSS phase 2400' has again three sub-phases: batchwise substance filtering 2410', substance reduction and importance weighting 2420', and result calculation 2430'. The steps performed in the EMSS phase are described in detail in the following in the context of FIG. 3 and FIG. 4B.

The batchwise substance filtering 2410' starts with composing 1510 a plurality of filter batches Batch_1 to Batch_N. Each filter batch the includes a top-ranked subset P of the predefined number M of reference spectra 291 (with P<<M) from the HIT list 290 selected in step 1410, as well as the reference spectra associated with the one or more substances provided by step 1460. The aggregate of these two sets of reference spectra included in each batch is referred to as "All Batch Substances" (All Batch Subst.) in the following. Further, the remaining reference spectra 311r from the library 300 (i.e., the reference spectra of the library which are not contained in the "All Batch Substances") are distributed among the filter batches (Z1-ZAA → Batch_1; ZAA-ZBB → Batch_2; ZXX-ZYY → Batch_N). The number of reference spectra per filter batch can be a fixed predefined number. That is, the number of filter batches depends on the number of reference spectra in the library to be distributed among the various filter batches. The reference spectra of each filter batch are then standardized

Module 140 then solves 1520 the optimization problem with the second set of hyperparameters (or with a similar set of hyperparameters with an emphasis on substance filtering) for each (standardized) filter batch/measure spectrum. As a result, for each filter batch, module 130 obtains the Top K substances associated with the respective batch input spectra which have the best match with the measured spectrum 211. The filter batches are all independent so that the processing of the filter batches is a parallelizable task which can be executed in parallel on a general-purpose computer having multiple cores.

Module 140 then consolidates 1530 the result sets RFB1 to RFBN of each filter batch by eliminating redundant substances from the consolidated result into a consolidated set of mixture substance candidates CMSC. In other words, the consolidated result being the concatenation of RFB1 to RFBN includes many redundant reference spectra (substances). The consolidated set of mixture substance candidates CMSC only includes each substance once - even if it occurred multiple times in said concatenation.

Module 140 then implements the subphases 2420' and 2430' by reapplying 1540 steps 1420 to 1460 while replacing the selected reference spectra of original step 1420 with the reference spectra associated with the consolidated set of mixture substance candidates CMSC.

FIG. 6 illustrates an example of dividing of a measured RAMAN spectrum 60pc into multiple analysis ranges $S_1$ to $S_3$ according to an embodiment. The example shows the same RAMAN mixture spectrum of Paracetamol and Caffeine as FIG. 5A. In the example, spectrum 60pc is divided into three analysis ranges separated by the dashed lines 60-1, 60-2.

In some cases (in particular in the case of RAMAN spectra), the spectra (measured and respective reference spectra) may be divided into R analysis ranges $S_i$ $i \in [1,R]$ to apply an intensity correction. Advantageously, the analysis ranges can be chosen such that the split 60-1, 60-2 occurs at a local minimum between peaks or groups of peaks in the measured spectrum and additional spectrum type specific information. A peak position can be identified with a peak pick method. The region around a peak can be defined by the full width at half maximum of the peak. If peaks are close to each other (threshold) they can be grouped together. In the example, the analysis ranges are chosen based on the information of peak locations in the measured spectrum and additional input spectrum type specific information. For example, RAMAN spectra typically show none or few spectral information in the so-called "silent region" around 1800 - 2600 $cm^{-1}$ wavenumbers (which is also true for the RAMAN spectrum 60pc). This information makes it beneficial to apply a split between analysis ranges in that region.

When applying an intensity correction to reference spectra to be matched to a measured RAMAN spectrum, it can be advantageous to apply such intensity corrections separately for each analysis region. In such cases of using multiple analysis ranges ($R > 1$), the respective hyperparameter is set to '1': $\mu = 1$.

If no intensity correction is applied (e.g., in the case of an IR spectrum) only a single analysis range is used ($R = 1$) and the respective hyperparameter is set to zero: $\mu = 0$.

In general, when solving the optimization problem for spectral corrections, the measured spectrum 60pc is divided into multiple analysis ranges $S_1$ to $S_3$ and one optimization problem is solved optimizing over all analysis ranges.

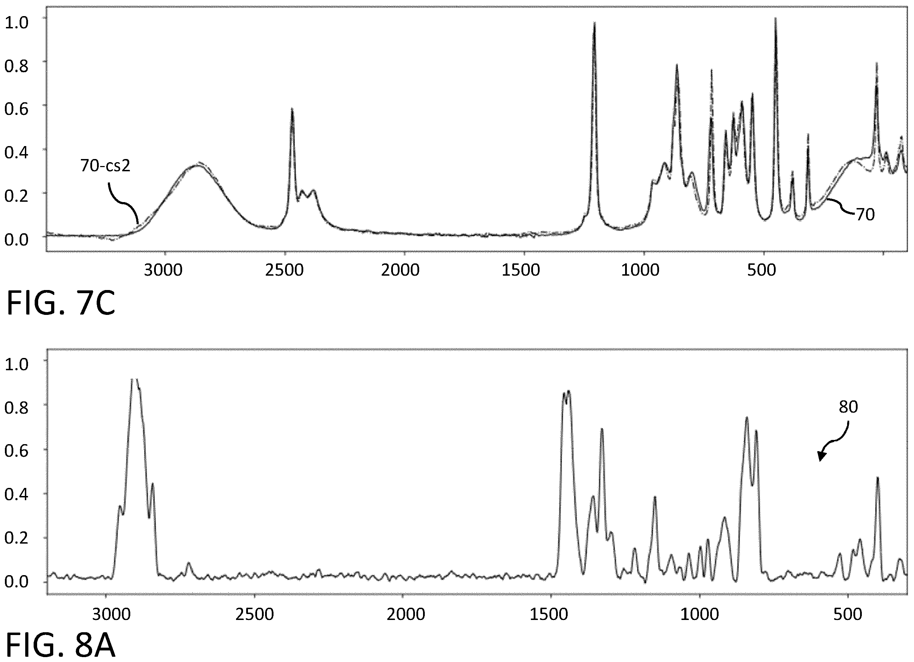

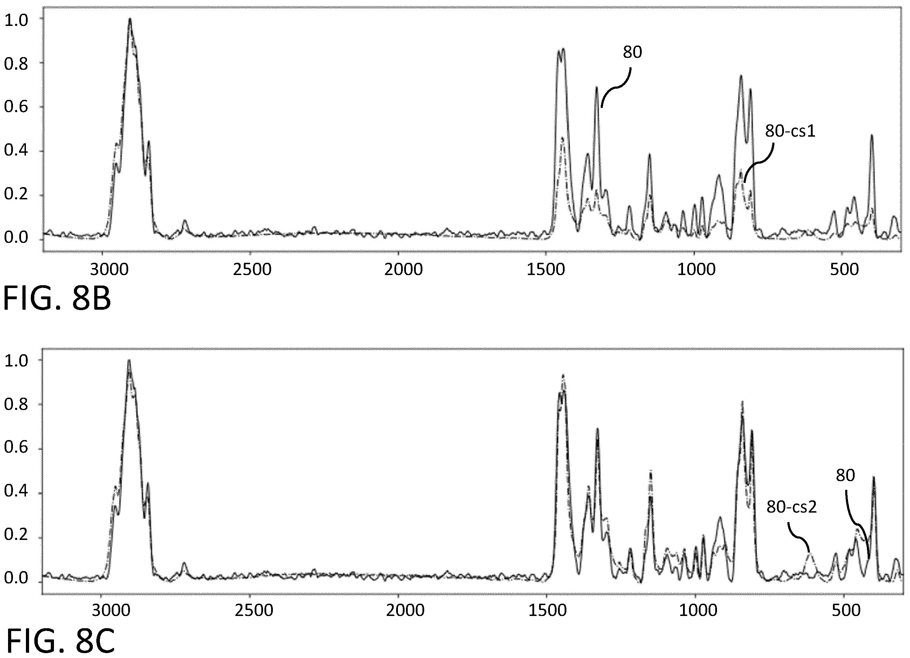

FIGs. 8A to 8C illustrate an example of intensity correction applied to a composite spectrum. In this example a mixture spectrum of EVOH (Ethylene vinyl alcohol) and PP (Polypropylene) is analyzed. The measured input spectrum 80 is shown in FIG. 8A. In FIG. 8B, the input spectrum 80 is shown in comparison with the composite spectrum 80cs-1 of the pure substance reference spectra of EVOH and PP (without intensity correction) as obtained from the library. A significant intensity distortion is visible between the two spectra (in particular for wavenumbers < 1500 $cm^{-1}$) which results in a L2 norm error of 3.774 and a correlation score of 0.87. FIG. 8C shows the input spectrum 80 compared to an adapted composite spectrum 80-cs2 (with intensity correction) of the pure substance reference spectra of EVOH and PP from the library. The intensity distortion is handled when solving the optimization problem with the first set of hyperparameters. Thereby, the L2 norm error is reduced to 1.549 and the correlation score increased to 0.97.

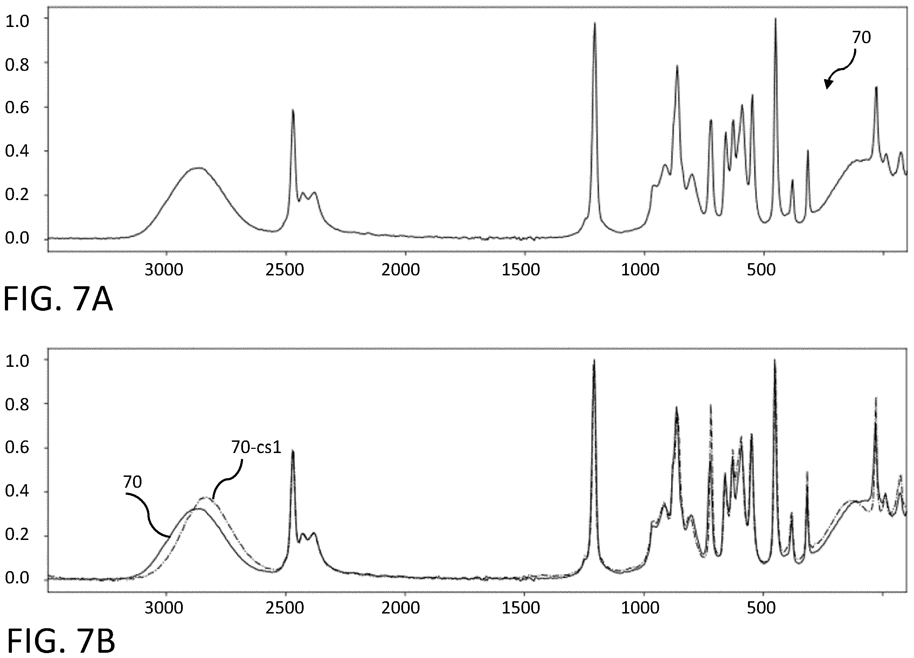

FIGs. 7A to 7C illustrate an example of peak shift correction in a composite spectrum. To apply a peak shift correction to a composite spectrum, peaks or peak clusters are identified in the input spectrum and their spectral ranges are denoted as $C_i$ for i $\in [1,P]$ where $P$ is the number of identified peak clusters.

For example, by identifying a peak with a peak pick method, one a region $C_i$ around that peak can be defined by the extracted peak position and the full width at half maximum information of the peak. If peaks are closer than a threshold to each other, they can be grouped into a peak cluster and thus define a single peak cluster range. In case that multiple analysis ranges are used, the analysis ranges $S_i$ may or may not overlap with the peak cluster ranges $C_i$.

In this example of FIG. 7A, a measured IR mixture spectrum 70 is shown. The respective sample contains Isopropanol, Ethanol and Acetone. Spectrum 70 is analyzed with the basic pipeline approach as described earlier by solving the presented optimization problem. In FIG. 7B, the measured mixture spectrum 70 is compared to a found composite spectrum 70-cs1 composed from the reference spectra of the pure substances Isopropanol, Ethanol and Acetone without a peak correction polynomial in the OH-stretch region C [3000-3800]. The peak shift of the OH-stretch is well visible for the peak at 2800 wavenumbers and the L2 norm error between the two spectra is 1.108. When the peak shift is not handled the risk increases of adding additional components from the reference library which may result in an incorrect artificial result for the sample composition. A peak comparison metric will also lead to larger errors in this case.

FIG. 7C shows the measure input mixture spectrum 70 compared to an adapted composite spectrum 70-cs2 composed from the reference spectra of the pure substances Isopropanol, Ethanol and Acetone with a peak correction polynomial in the OH-stretch region. The peak shift of the OH-stretch is now significantly reduced and the L2 norm error between the two spectra is reduced to 0.85.

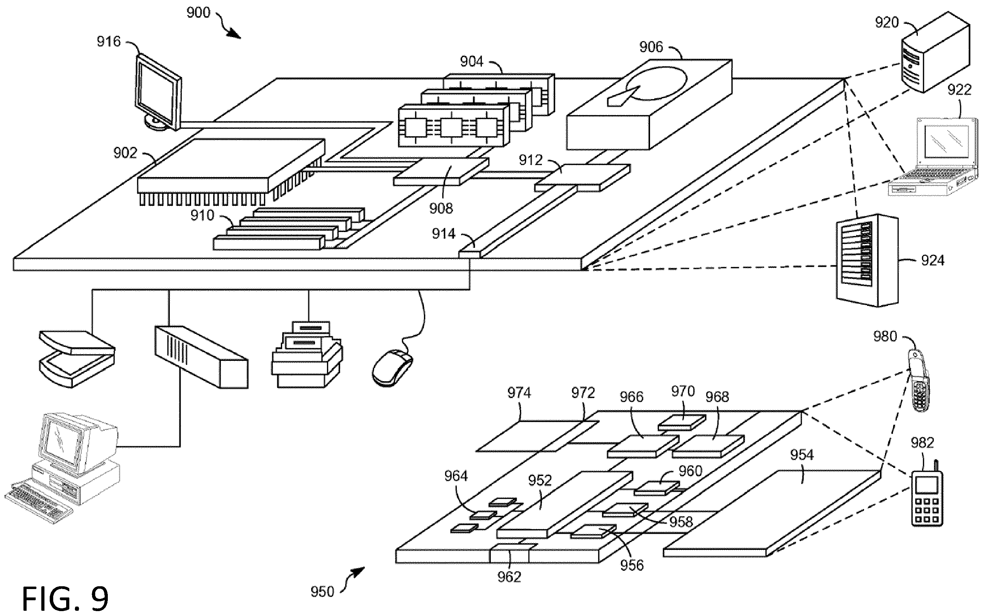

FIG. 9 is a diagram that shows an example of a generic computer device 900 and a generic mobile computer device 950, which may be used with the techniques described here. In some embodiments, computing device 900 may relate to system 100 (cf. FIG. 1). Computing device 950 is intended to represent various forms of mobile devices, such as personal digital assistants, cellular telephones, smart phones, and other similar computing devices. In the context of this disclosure the computing device 950 may provide I/O means for a user to interact with the computing device 900. For example, device 950 may be used to provide results obtained by device 900 to a user. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low-speed interface 912 connecting to low-speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high-speed interface 908. In other implementations, multiple processors and/or multiple buses may be used, as appropriate, along with multiple memories and types of memory. Also, multiple computing devices 900 may be connected, with each device providing portions of the necessary operations (e.g., as a server bank, a group of blade servers, or a multi-processor system).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high-speed controller 908 manages bandwidth-intensive operations for the computing device 900, while the low-speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processors. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provide in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952, that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

## Claims

1. A computer-implemented method (1000) for identifying substances contained in a sample (201) based on a measured spectrum (211) obtained from the sample via an optical spectroscopy method, comprising:

   obtaining (1100) the measured spectrum (211), and accessing at least one library (300) comprising a plurality of reference spectra (311*) of known pure substances;

   pre-processing (1200) the measured spectrum and the plurality of reference spectra by applying a min-max normalization to each spectrum such that the intensity ranges of all spectra are within the interval [0, 1], and ensuring that all spectra have the same spectral range with the same spectral resolution;

   performing (1300) a single substance search by:

   a) solving (1310) an optimization problem with a first set of hyperparameters for all possible pairs of the measured spectrum with the reference spectra in the library, the optimization problem being formulated as a single optimization problem for regression, substance filtering and spectral corrections, wherein spectral corrections comprise corrections of peak shift and baseline, and in case of RAMAN spectroscopy, also comprise corrections of intensity distortion, the first set of hyperparameters being configured to optimally adapt the reference spectra to the measured spectrum for a spectral comparison; and

   b) determining (1320) a similarity score for each pair of measured spectrum and adapted reference spectrum using a similarity metric scoring the spectral match of two spectra, and ranking the corresponding reference spectra in accordance with the similarity scores of the respective adapted reference spectra in a HIT list (290);

   performing (1400) a limited multi-substance search by:

   c) selecting (1410) a predefined number M of reference spectra from the HIT list (290) starting with the reference spectrum having the highest similarity score, and standardizing the selected reference spectra;

   d) solving (1420) the optimization problem with a second set of hyperparameters with the measured spectrum and all standardized reference spectra, wherein the second set of hyperparameters being configured such that substance filtering is emphasized to result in a set of mixture substance candidates;

   e) solving (1430) the optimization problem with a third set of hyperparameters with the measured spectrum and the N standardized reference spectra associated with the mixture substance candidates, wherein the third set of hyperparameters being configured such that the filtering effect is attenuated in relation to the second set of hyperparameters whereby substances are discarded until all remaining P substances have a relative weight greater than a predefined threshold of significance, the relative weight being the normalized weight of the result of the optimization problem;

   f) computing (1440) a plurality of composition batches from the normalized reference spectra associated with the P remaining substances such that a composition batch is generated for each possible subset of the P remaining substances which includes the remaining substance with the highest relative weight;

   g) solving (1450) the optimization problem for each composition batch and the measured spectrum with the first set of hyperparameters resulting in a composite spectrum per composition batch; and

   h) evaluating (1460) the quality of each composite spectrum by evaluating the results of the optimization problem, as obtained by using the first set of hyperparameters, with said similarity metric scoring the spectral match of the respective composite spectrum with the measured spectrum, and providing substances associated with composite spectra having a quality above a predefined quality threshold as set of substances (R2) contained in the sample.

2. The method of claim 1, further comprising:
performing (1500) an extended multi-substance search by:

i) composing (1510) a plurality of filter batches wherein each filter batch includes a top-ranked subset P of the predefined number M of reference spectra from the HIT list (290) of step c), the reference spectra associated with the one or more substances provided by step h), and a predefined number of the remaining reference spectra from the library (300), and standardizing the reference spectra of each filter batch;
j) solving (1520) the optimization problem with the second set of hyperparameters for each filter batch (Batch_1 to Batch_N) and the measured spectrum (211);
k) consolidating (1530) the result sets of each filter batch by eliminating redundant substances from the consolidated result into a consolidated set of mixture substance candidates; and
l) reapplying (1540) steps d) to h) by using the reference spectra associated with the consolidated set of mixture substance candidates instead of the selected reference spectra of step d).

3. The method of any of the previous claims, wherein the optical spectroscopy method is selected from any of: RAMAN spectroscopy, infrared spectroscopy, and quantum-cascade laser spectroscopy.

4. The method of any of the previous claims, wherein the similarity metric is a weighted average of a fit similarity metric and a peak similarity metric.

5. The method of any of the previous claims, wherein in step d), the size N of the set of mixture substance candidates is at least one order of magnitude smaller than the number M of selected reference spectra from the HIT list.

6. The method of any of the previous claims, wherein in step f), computing (1440) the plurality of composite spectra is performed in a series of composition batches, wherein each composition batch contains subsets with the same number of substances, and wherein in step g), the optimization problem is solved (1450) for each composition batch.

7. The method of any of the previous claims, wherein, when solving the optimization problem for spectral corrections, the measured spectrum (60pc) is divided into multiple analysis ranges ($S_1$ to $S_3$) and one optimization problem is solved optimizing over all analysis ranges.

8. The method of claim 7, wherein the analysis ranges are chosen such that a split (60-1, 60-2) between two adjacent analysis ranges occurs at a local minimum between peaks or groups of peaks in the measured spectrum (211).

9. A computer program product for identifying one or more substances contained in a sample (201) based on a measured spectrum (211) obtained from the sample via an optical spectroscopy method, comprising computer-readable instructions that, when loaded into a memory of a computing device and executed by one or more processors of the computing device, cause the computing device to execute the steps of the computer-implemented method according to any of the previous claims.

10. A computer system (100) for identifying substances contained in a sample (201) based on a measured spectrum (211) obtained from the sample via optical spectroscopy, the system comprising:

an interface configured to obtain the measured spectrum (211), and to access at least one library (300) comprising a plurality of reference spectra (311*) of known pure substances;
a pre-processing module 110 configured to pre-process the measured spectrum and the plurality of reference spectra by applying a min-max normalization to each spectrum such that the intensity ranges of all spectra are within the interval [0, 1], and to ensure that all spectra have the same spectral range with the same spectral resolution;
a single substance search module (120) configured to perform a single substance search by:

m) solving an optimization problem with a first set of hyperparameters for all possible pairs of the measured spectrum with the reference spectra in the library, the optimization problem being formulated as a single optimization problem for regression, substance filtering and spectral corrections, wherein spectral corrections comprise corrections of peak shift and baseline, and in case of RAMAN spectroscopy, also comprise corrections of intensity distortion, the first set of hyperparameters being configured to optimally adapt the reference spectra to the measured spectrum for a spectral comparison; and
n) determining a similarity score for each pair of measured spectrum and adapted reference spectrum using a similarity metric scoring the spectral match of two spectra, and ranking the corresponding reference spectra in accordance with the similarity scores of the respective adapted reference spectra in a HIT list (290);

a limited multi-substance search module (130) configured to performing a limited multi-substance search by:

o) selecting a predefined number M of reference spectra from the HIT list (290) starting with the reference spectrum having the highest similarity score and standardizing the selected reference spectra;

p) solving the optimization problem with a second set of hyperparameters with the measured spectrum and all standardized reference spectra, wherein the second set of hyperparameters being configured such that substance filtering is emphasized to result in a set of mixture substance candidates;

q) solving the optimization problem with a third set of hyperparameters with the measured spectrum and the N standardized reference spectra associated with the mixture substance candidates, wherein the third set of hyperparameters being configured such that the filtering effect is attenuated in relation to the second set of hyperparameters whereby substances are discarded until all remaining P substances have a relative weight greater than a predefined threshold of significance, the relative weight being the normalized weight of the result of the optimization problem;

r) computing a plurality of composition batches from the normalized reference spectra associated with the P remaining substances such that a composition batch is generated for each possible subset of the P remaining substances which includes the remaining substance with the highest relative weight;

s) solving the optimization problem for each composition batch and the measured spectrum with the first set of hyperparameters resulting in a composite spectrum per composition batch; and

t) evaluating the quality of each composite spectrum by evaluating the results of the optimization problem, as obtained by using the first set of hyperparameters, with said similarity metric scoring the spectral match of the respective composite spectrum with the measured spectrum, and providing substances associated with composite spectra having a quality above a predefined quality threshold as set of substances (R2) contained in the sample.

11. The system of claim 10, further comprising:
an extended multi-substance search module (140) configured to perform an extended multi-substance search by:

u) composing a plurality of filter batches wherein each filter batch includes a top-ranked subset P of the predefined number M of reference spectra from the HIT list (290) of step o), the reference spectra associated with the one or more substances provided by step t), and a predefined number of the remaining reference spectra from the library (300), and standardizing the reference spectra of each filter batch;

v) solving the optimization problem with the second set of hyperparameters for each filter batch (Batch_1 to Batch_N) and the measured spectrum (211);

w) consolidating the result sets of each filter batch by eliminating redundant substances from the consolidated result into a consolidated set of mixture substance candidates; and

x) reapplying steps p) to t) by using the reference spectra associated with the consolidated set of mixture substance candidates instead of the selected reference spectra of step p).

12. The system of any of claim 10 or 11, wherein the optical spectroscopy is selected from any of: RAMAN spectroscopy, infrared spectroscopy, and quantum-cascade laser spectroscopy

13. The system of any of the claims 10 to 12, wherein the similarity metric is a weighted average of a fit similarity metric and a peak similarity metric.

**Patentansprüche**

1. Computer-implementiertes Verfahren (1000) zum Identifizieren von Stoffen, die in einer Probe (201) enthalten sind, basierend auf einem gemessenen Spektrum (211), das aus der Probe über ein optisches Spektroskopieverfahren erhalten wird, umfassend:

Erhalten (1100) des gemessenen Spektrums (211) und Zugreifen auf mindestens eine Bibliothek (300), umfassend eine Vielzahl von Referenzspektren (311*) bekannter reiner Stoffe;
Vorverarbeiten (1200) des gemessenen Spektrums und der Vielzahl von Referenzspektren durch Anwenden einer Min-Max-Normalisierung auf jedes Spektrum, sodass die Intensitätsbereiche aller Spektren innerhalb des Intervalls [0, 1] liegen, und Sicherstellen, dass alle Spektren den gleichen Spektralbereich mit der gleichen spektralen Auflösung aufweisen;

Durchführen (1300) einer Einzelstoffsuche durch:

a) Lösen (1310) eines Optimierungsproblems mit einem ersten Satz von Hyperparametern für alle möglichen Paare des gemessenen Spektrums mit den Referenzspektren in der Bibliothek, wobei das Optimierungsproblem als ein einzelnes Optimierungsproblem für Regression, Stofffilterung und spektrale Korrekturen formuliert ist, wobei spektrale Korrekturen Korrekturen einer Spitzenverschiebung und Basislinie umfassen und im Falle einer RAMAN-Spektroskopie auch Korrekturen einer Intensitätsverzerrung umfassen, wobei der erste Satz von Hyperparametern konfiguriert ist, um die Referenzspektren für einen spektralen Vergleich an das gemessene Spektrum optimal anzupassen; und

b) Bestimmen (1320) einer Ähnlichkeitsbewertung für jedes Paar aus gemessenem Spektrum und angepasstem Referenzspektrum unter Verwendung einer Ähnlichkeitsmetrik, die die spektrale Übereinstimmung zweier Spektren bewertet, und Einordnen der entsprechenden Referenzspektren gemäß den Ähnlichkeitsbewertungen der jeweiligen angepassten Referenzspektren in einer HIT-Liste (290);

Durchführen (1400) einer eingeschränkten Mehrstoffsuche durch:

c) Auswählen (1410) einer vordefinierten Anzahl M von Referenzspektren aus der HIT-Liste (290), beginnend mit dem Referenzspektrum, das die höchste Ähnlichkeitsbewertung aufweist, und Standardisieren der ausgewählten Referenzspektren;

d) Lösen (1420) des Optimierungsproblems mit einem zweiten Satz von Hyperparametern mit dem gemessenen Spektrum und allen standardisierten Referenzspektren, wobei der zweite Satz von Hyperparametern konfiguriert ist, sodass die Stofffilterung betont wird, um einen Satz von Mischungsstoffkandidaten zu ergeben;

e) Lösen (1430) des Optimierungsproblems mit einem dritten Satz von Hyperparametern mit dem gemessenen Spektrum und den N standardisierten Referenzspektren, die den Mischungsstoffkandidaten zugeordnet sind, wobei der dritte Satz von Hyperparametern konfiguriert ist, sodass der Filterungseffekt im Verhältnis zu dem zweiten Satz von Hyperparametern abgeschwächt wird, wodurch Stoffe verworfen werden, bis alle verbleibenden P Stoffe ein relatives Gewicht aufweisen, das größer als ein vordefinierter Signifikanzschwellenwert ist, wobei das relative Gewicht das normalisierte Gewicht des Ergebnisses des Optimierungsproblems ist;

f) Berechnen (1440) einer Vielzahl von Zusammensetzungschargen aus den normalisierten Referenzspektren, die den P verbleibenden Stoffen zugeordnet sind, sodass eine Zusammensetzungscharge für jeden möglichen Untersatz der P verbleibenden Stoffen erzeugt wird, die den verbleibenden Stoff mit dem höchsten relativen Gewicht einschließt;

g) Lösen (1450) des Optimierungsproblems für jede Zusammensetzungscharge und das gemessene Spektrum mit dem ersten Satz von Hyperparametern, was ein zusammengesetztes Spektrum pro Zusammensetzungscharge ergibt; und

h) Evaluieren (1460) der Qualität jedes zusammengesetzten Spektrums durch Evaluieren der Ergebnisse des Optimierungsproblems, wie sie durch Verwenden des ersten Satzes von Hyperparametern erhalten werden, wobei die Ähnlichkeitsmetrik die spektrale Übereinstimmung des jeweiligen zusammengesetzten Spektrums mit dem gemessenen Spektrum bewertet und Stoffe, die zusammengesetzten Spektren zugeordnet sind, die eine Qualität über einem vordefinierten Qualitätsschwellenwert aufweisen, als einen Satz von Stoffen (R2), der in der Probe enthalten ist, bereitstellt.

2. Verfahren nach Anspruch 1, ferner umfassend:
Durchführen (1500) einer erweiterten Mehrstoffsuche durch:

i) Zusammensetzen (1510) einer Vielzahl von Filterchargen, wobei jede Filtercharge einen an oberster Stelle eingeordneten Untersatz P der vordefinierten Anzahl M von Referenzspektren aus der HIT-Liste (290) von Schritt c), die Referenzspektren, die dem einen oder den mehreren durch Schritt h) bereitgestellten Stoffen zugeordnet sind, und eine vordefinierte Anzahl der verbleibenden Referenzspektren aus der Bibliothek (300) einschließt, und Standardisieren der Referenzspektren jeder Filtercharge;

j) Lösen (1520) des Optimierungsproblems mit dem zweiten Satz von Hyperparametern für jede Filtercharge (Batch_1 bis Batch_N) und das gemessene Spektrum (211);

k) Konsolidieren (1530) der Ergebnissätze jeder Filtercharge durch Eliminieren redundanter Stoffe aus dem konsolidierten Ergebnis in einen konsolidierten Satz von Mischungsstoffkandidaten; und

l) erneutes Anwenden (1540) der Schritte d) bis h) durch Verwenden der Referenzspektren, die dem konsolidierten Satz von Mischungsstoffkandidaten zugeordnet sind, anstelle der ausgewählten Referenzspektren von Schritt d).

3. Verfahren nach einem der vorstehenden Ansprüche, wobei das optische Spektroskopieverfahren aus einem beliebigen ausgewählt ist von: RAMAN-Spektroskopie, Infrarotspektroskopie und Quantenkaskadenlaserspektroskopie.

4. Verfahren nach einem der vorstehenden Ansprüche, wobei die Ähnlichkeitsmetrik ein gewichteter Durchschnitt einer Passähnlichkeitsmetrik und einer Spitzenähnlichkeitsmetrik ist.

5. Verfahren nach einem der vorstehenden Ansprüche, wobei in Schritt d) die Größe N des Satzes von Mischungsstoffkandidaten mindestens eine Größenordnung kleiner als die Anzahl M von ausgewählten Referenzspektren aus der HIT-Liste ist.

6. Verfahren nach einem der vorstehenden Ansprüche, wobei in Schritt f) das Berechnen (1440) der Vielzahl von zusammengesetzten Spektren in einer Reihe von Zusammensetzungschargen durchgeführt wird, wobei jede Zusammensetzungscharge Untersätze mit der gleichen Anzahl von Stoffen enthält, und wobei in Schritt g) das Optimierungsproblem für jede Zusammensetzungscharge gelöst (1450) wird.

7. Verfahren nach einem der vorstehenden Ansprüche, wobei, wenn das Optimierungsproblem für spektrale Korrekturen gelöst wird, das gemessene Spektrum (60pc) in mehrere Analysebereiche ($S_1$ bis $S_3$) unterteilt wird und ein Optimierungsproblem durch Optimieren über alle Analysebereiche gelöst wird.

8. Verfahren nach Anspruch 7, wobei die Analysebereiche gewählt werden, sodass eine Aufteilung (60-1, 60- 2) zwischen zwei benachbarten Analysebereichen an einem lokalen Minimum zwischen Spitzen oder Spitzengruppen in dem gemessenen Spektrum (211) auftritt.

9. Computer-Programm-Produkt zum Identifizieren eines oder mehrerer Stoffe, die in einer Probe (201) enthalten sind, basierend auf einem gemessenen Spektrum (211), das von der Probe über ein optisches Spektroskopieverfahren erhalten wird, umfassend computer-lesbare Anweisungen, die, wenn sie in einen Speicher einer Computer-Vorrichtung geladen und durch einen oder mehrere Prozessoren der Computer-Vorrichtung ausgeführt werden, die Computer-Vorrichtung veranlassen, die Schritte des computer-implementierten Verfahrens nach einem der vorstehenden Ansprüche auszuführen.

10. Computer-System (100) zum Identifizieren von Stoffen, die in einer Probe (201) enthalten sind, basierend auf einem gemessenen Spektrum (211), das aus der Probe über optische Spektroskopie erhalten wird, das System umfassend:

eine Schnittstelle, die konfiguriert ist, um das gemessene Spektrum (211) zu erhalten und um auf mindestens eine Bibliothek (300) zuzugreifen, umfassend eine Vielzahl von Referenzspektren (311*) bekannter reiner Stoffe; ein Vorverarbeitungsmodul 110, das konfiguriert ist, um das gemessene Spektrum und die Vielzahl von Referenzspektren durch Anwenden einer Min-Max-Normalisierung auf jedes Spektrum vorzuverarbeiten, sodass die Intensitätsbereiche aller Spektren innerhalb des Intervalls [0, 1] liegen, und um sicherzustellen, dass alle Spektren den gleichen Spektralbereich mit der gleichen spektralen Auflösung aufweisen; ein Einzelstoffsuchmodul (120), das konfiguriert ist, um eine Einzelstoffsuche durchzuführen durch:

m) Lösen eines Optimierungsproblems mit einem ersten Satz von Hyperparametern für alle möglichen Paare des gemessenen Spektrums mit den Referenzspektren in der Bibliothek, wobei das Optimierungsproblem als ein einzelnes Optimierungsproblem für Regression, Stofffilterung und spektrale Korrekturen formuliert ist, wobei spektrale Korrekturen Korrekturen der Spitzenverschiebung und Basislinie umfassen und im Falle der RAMAN-Spektroskopie auch Korrekturen der Intensitätsverzerrung umfassen, wobei der erste Satz von Hyperparametern konfiguriert ist, um die Referenzspektren für einen spektralen Vergleich an das gemessene Spektrum optimal anzupassen; und
n) Bestimmen einer Ähnlichkeitsbewertung für jedes Paar aus gemessenem Spektrum und angepasstem Referenzspektrum unter Verwendung einer Ähnlichkeitsmetrik, die die spektrale Übereinstimmung zweier Spektren bewertet, und Einordnen der entsprechenden Referenzspektren gemäß den Ähnlichkeitsbewertungen der jeweiligen angepassten Referenzspektren in einer HIT-Liste (290);

ein Modul (130) für die eingeschränkte Mehrstoffsuche, das konfiguriert ist, um eine eingeschränkte Mehrstoffsuche durchzuführen durch:

o) Auswählen einer vordefinierten Anzahl M von Referenzspektren aus der HIT-Liste (290), beginnend mit dem Referenzspektrum, das die höchste Ähnlichkeitsbewertung aufweist, und Standardisieren der ausgewählten Referenzspektren;

p) Lösen des Optimierungsproblems mit einem zweiten Satz von Hyperparametern mit dem gemessenen Spektrum und allen standardisierten Referenzspektren, wobei der zweite Satz von Hyperparametern konfiguriert ist, sodass die Stofffilterung betont wird, um einen Satz von Mischungsstoffkandidaten zu ergeben;

q) Lösen des Optimierungsproblems mit einem dritten Satz von Hyperparametern mit dem gemessenen Spektrum und den N standardisierten Referenzspektren, die den Mischungsstoffkandidaten zugeordnet sind, wobei der dritte Satz von Hyperparametern konfiguriert ist, sodass der Filterungseffekt im Verhältnis zu dem zweiten Satz von Hyperparametern abgeschwächt wird, wodurch Stoffe verworfen werden, bis alle verbleibenden P Stoffe ein relatives Gewicht aufweisen, das größer als ein vordefinierter Signifikanzschwellenwert ist, wobei das relative Gewicht das normalisierte Gewicht des Ergebnisses des Optimierungsproblems ist;

r) Berechnen einer Vielzahl von Zusammensetzungschargen aus den normalisierten Referenzspektren, die den P verbleibenden Stoffen zugeordnet sind, sodass eine Zusammensetzungscharge für jeden möglichen Untersatz der P verbleibenden Stoffen erzeugt wird, die den verbleibenden Stoff mit dem höchsten relativen Gewicht einschließt;

s) Lösen des Optimierungsproblems für jede Zusammensetzungscharge und das gemessene Spektrum mit dem ersten Satz von Hyperparametern, was ein zusammengesetztes Spektrum pro Zusammensetzungscharge ergibt; und

t) Evaluieren der Qualität jedes zusammengesetzten Spektrums durch Evaluieren der Ergebnisse des Optimierungsproblems, wie sie durch Verwenden des ersten Satzes von Hyperparametern erhalten werden, wobei die Ähnlichkeitsmetrik die spektrale Übereinstimmung des jeweiligen zusammengesetzten Spektrums mit dem gemessenen Spektrum bewertet und Stoffe, die zusammengesetzten Spektren zugeordnet sind, die eine Qualität über einem vordefinierten Qualitätsschwellenwert aufweisen, als einen Satz von Stoffen (R2), der in der Probe enthalten ist, bereitstellt.

11. System nach Anspruch 10, ferner umfassend:
ein Modul (140) für die erweiterte Mehrstoffsuche, das konfiguriert ist, um eine erweiterte Mehrstoffsuche durchzuführen durch:

u) Zusammensetzen einer Vielzahl von Filterchargen, wobei jede Filtercharge einen an oberster Stelle eingeordneten Untersatz P der vordefinierten Anzahl M von Referenzspektren aus der HIT-Liste (290) von Schritt o), die Referenzspektren, die dem einen oder den mehreren durch Schritt t) bereitgestellten Stoffen zugeordnet sind, und eine vordefinierte Anzahl der verbleibenden Referenzspektren aus der Bibliothek (300) einschließt, und Standardisieren der Referenzspektren jeder Filtercharge;

v) Lösen des Optimierungsproblems mit dem zweiten Satz von Hyperparametern für jede Filtercharge (Batch_1 bis Batch_N) und das gemessene Spektrum (211);

w) Konsolidieren der Ergebnissätze jeder Filtercharge durch Eliminieren redundanter Stoffe aus dem konsolidierten Ergebnis in einen konsolidierten Satz von Mischungsstoffkandidaten; und

x) erneutes Anwenden der Schritte p) bis t) durch Verwenden der Referenzspektren, die dem konsolidierten Satz von Mischungsstoffkandidaten zugeordnet sind, anstelle der ausgewählten Referenzspektren von Schritt p).

12. System nach einem von Anspruch 10 oder 11, wobei die optische Spektroskopie aus einer ausgewählt ist von: RAMAN-Spektroskopie, Infrarotspektroskopie und Quantenkaskadenlaserspektroskopie

13. System nach einem der Ansprüche 10 bis 12, wobei die Ähnlichkeitsmetrik ein gewichteter Durchschnitt einer Passähnlichkeitsmetrik und einer Spitzenähnlichkeitsmetrik ist.

**Revendications**

1. Procédé mis en œuvre par ordinateur (1000) permettant d'identifier des substances contenues dans un échantillon (201) en fonction d'un spectre mesuré (211) obtenu à partir de l'échantillon par le biais d'un procédé de spectroscopie optique, comprenant :

l'obtention (1100) du spectre mesuré (211), et l'accès à au moins une bibliothèque (300) comprenant une pluralité de spectres de référence (311*) de substances pures connues ;

le prétraitement (1200) du spectre mesuré et de la pluralité de spectres de référence en appliquant une normalisation min-max à chaque spectre de telle sorte que les plages d'intensité de tous les spectres sont au sein de l'intervalle [0, 1], et en veillant à ce que tous les spectres aient la même plage spectrale avec la même résolution spectrale ;

la réalisation (1300) d'une recherche de substances unique en :

a) résolvant (1310) un problème d'optimisation avec un premier ensemble d'hyperparamètres pour toutes les paires possibles du spectre mesuré avec les spectres de référence dans la bibliothèque, le problème d'optimisation étant formulé en guise de problème d'optimisation unique pour la régression, le filtrage de substances et les corrections spectrales, dans lequel les corrections spectrales comprennent des corrections de décalage de pics et de ligne de base, et dans le cas d'une spectroscopie RAMAN, comprennent également des corrections de distorsion d'intensité, le premier ensemble d'hyperparamètres étant configuré pour adapter de manière optimale les spectres de référence au spectre mesuré pour une comparaison spectrale ; et

b) déterminant (1320) un score de similarité pour chaque paire de spectre mesuré et de spectre de référence adapté à l'aide d'un score métrique de similarité évaluant la correspondance spectrale de deux spectres, et classant les spectres de référence correspondants conformément aux scores de similarité des spectres de référence adaptés respectifs dans une liste HIT (290) ;

la réalisation (1400) d'une recherche multi-substances limitée en :

c) sélectionnant (1410) un nombre prédéfini M de spectres de référence à partir de la liste HIT (290), en commençant avec le spectre de référence ayant le score de similarité le plus élevé, et normalisant les spectres de référence sélectionnés ;

d) résolvant (1420) le problème d'optimisation avec un deuxième ensemble d'hyperparamètres avec le spectre mesuré et tous les spectres de référence normalisés, dans lequel le deuxième ensemble d'hyperparamètres étant configuré de telle sorte que le filtrage de substances est accentué pour résulter en un ensemble de substances candidates de mélange ;

e) résolvant (1430) le problème d'optimisation avec un troisième ensemble d'hyperparamètres avec le spectre mesuré et les N spectres de référence normalisés associés aux substances candidates de mélange, dans lequel le troisième ensemble d'hyperparamètres étant configuré de telle sorte que l'effet de filtrage est atténué par rapport au deuxième ensemble d'hyperparamètres moyennant quoi

les substances sont éliminées jusqu'à ce que toutes les P substances restantes
aient un poids relatif supérieur à un seuil d'importance prédéfini, le poids relatif étant le poids normalisé du résultat du problème d'optimisation ;

f) calculant (1440) une pluralité de lots de composition à partir des spectres de référence normalisés associés aux P substances restantes de telle sorte qu'un lot de composition est généré pour chaque sous-ensemble possible des P substances restantes qui comporte la substance restante avec le poids relatif le plus élevé ;

g) résolvant (1450) le problème d'optimisation pour chaque lot de composition et le spectre mesuré avec le premier ensemble d'hyperparamètres résultant en un spectre composite par lot de composition ; et

h) évaluant (1460) la qualité de chaque spectre composite en évaluant les résultats du problème d'optimisation, tels qu'obtenus à l'aide du premier ensemble d'hyperparamètres, avec ladite métrique de similarité évaluant la correspondance spectrale du spectre composite respectif avec le spectre mesuré, et fournissant des substances associées aux spectres composites ayant une qualité supérieure à un seuil de qualité prédéfini en guise d'ensemble de substances (R2) contenues dans l'échantillon.

2. Procédé selon la revendication 1, comprenant en outre :
la réalisation (1500) d'une recherche multi-substances étendue en :

i) composant (1510) une pluralité de lots de filtre dans lequel chaque lot de filtre comporte un sous-ensemble P de premier rang du nombre prédéfini M de spectres de référence à partir de la liste HIT (290) de l'étape c), les spectres de référence associés à la une ou plusieurs substances fournies par l'étape h), et un nombre prédéfini des spectres de référence restants à partir de la bibliothèque (300), et normalisant les spectres de référence de chaque lot de filtre ;

j) résolvant (1520) le problème d'optimisation avec le deuxième ensemble d'hyperparamètres pour chaque lot de filtre (Lot_I à Lot_N) et le spectre mesuré (211) ;

k) consolidant (1530) les ensembles de résultats de chaque lot de filtre en éliminant les substances redondantes du résultat consolidé dans un ensemble consolidé de substances candidates de mélange ; et

l) réappliquant (1540) les étapes d) à h) à l'aide des spectres de référence associés à l'ensemble consolidé de substances candidates de mélange au lieu des spectres de référence sélectionnés de l'étape d).

3. Procédé selon l'une quelconque des revendications précédentes, dans lequel le modèle de spectroscopie optique est choisi parmi l'un quelconque de : spectroscopie RAMAN, spectroscopie infrarouge, et spectroscopie laser à cascade quantique.

4. Procédé selon l'une quelconque des revendications précédentes, dans lequel la métrique de similarité est une moyenne pondérée d'une métrique de similarité d'ajustement et d'une métrique de similarité de pic.

5. Procédé selon l'une quelconque des revendications précédentes, dans lequel dans l'étape d), la taille N de l'ensemble de substances candidates de mélange est au moins d'un ordre de grandeur inférieur au nombre M de spectres de référence sélectionnés à partir de la liste HIT.

6. Procédé selon l'une quelconque des revendications précédentes, dans lequel dans l'étape f), le calcul (1440) de la pluralité de spectres composites est effectué dans une série de lots de composition, dans lequel chaque lot de composition contient des sous-ensembles avec le même nombre de substances, et dans lequel dans l'étape g), le problème d'optimisation est résolu (1450) pour chaque lot de composition.

7. Procédé selon l'une quelconque des revendications précédentes, dans lequel, lors de la résolution du problème d'optimisation pour des corrections spectrales, le spectre mesuré (60pc) est divisé en de multiples plages d'analyse ($S_1$ à $S_3$) et un problème d'optimisation est résolu en optimisant toutes les plages d'analyse.

8. Procédé selon la revendication 7, dans lequel les plages d'analyse sont choisies de telle sorte qu'une division (60-1, 60-2) entre deux plages d'analyse adjacentes se produit à un minimum local entre des pics ou des groupes de pics dans le spectre mesuré (211).

9. Produit programme d'ordinateur permettant d'identifier une ou plusieurs substances contenues dans un échantillon (201) en fonction d'un spectre mesuré (211) obtenu à partir de l'échantillon par le biais d'un procédé de spectroscopie optique, comprenant des instructions lisibles par ordinateur qui, lorsque chargées dans une mémoire d'un dispositif informatique et exécutées par un ou plusieurs processeurs du dispositif informatique, amènent le dispositif informatique à exécuter les étapes du procédé mis en œuvre par ordinateur selon l'une quelconque des revendications précédentes.

10. Système informatique (100) permettant d'identifier des substances contenues dans un échantillon (201) en fonction d'un spectre mesuré (211) obtenu à partir de l'échantillon par le biais d'une spectroscopie optique, le système comprenant :

une interface configurée pour obtenir le spectre mesuré (211), et pour accéder à au moins une bibliothèque (300) comprenant une pluralité de spectres de référence (311*) de substances pures connues ;

un module de prétraitement 110 configuré pour prétraiter le spectre mesuré et la pluralité de spectres de référence en appliquant une normalisation min-max à chaque spectre de telle sorte que les plages d'intensité de tous les spectres sont au sein de l'intervalle [0, 1], et pour s'assurer que tous les spectres ont la même plage spectrale avec la même résolution spectrale ;

un module de recherche de substance unique (120) configuré pour effectuer une recherche de substance unique par :

m) résolution d'un problème d'optimisation avec un premier ensemble d'hyperparamètres pour toutes les paires possibles du spectre mesuré avec les spectres de référence dans la bibliothèque, le problème d'optimisation étant formulé en guise de problème d'optimisation unique pour la régression, le filtrage de substances et les corrections spectrales, dans lequel les corrections spectrales comprennent des corrections de décalage de pics et de ligne de base, et dans le cas d'une spectroscopie RAMAN, comprennent également des corrections de distorsion d'intensité, le premier ensemble d'hyperparamètres étant configuré pour adapter de manière optimale les spectres de référence au spectre mesuré pour une comparaison spectrale ; et

n) détermination d'un score de similarité pour chaque paire de spectre mesuré et de spectre de référence adapté à l'aide d'une métrique de similarité évaluant la correspondance spectrale de deux spectres, et classant les spectres de référence correspondants conformément aux scores de similarité des spectres de référence adaptés respectifs dans une liste HIT (290) ;

un module de recherche multi-substances limitée (130) configuré pour effectuer une recherche multi-substances limitée en :

o) sélectionnant un nombre prédéfini M de spectres de référence à partir de la liste HIT (290) en commençant avec le spectre de référence ayant le score de similarité le plus élevé et normalisant les spectres de référence sélectionnés ;

p) résolvant le problème d'optimisation avec un deuxième ensemble d'hyperparamètres avec le spectre mesuré et tous les spectres de référence normalisés, dans lequel le deuxième ensemble d'hyperparamètres étant configuré de telle sorte que le filtrage de substances est accentué pour résulter en un ensemble de substances candidates de mélange ;

q) résolvant le problème d'optimisation avec un troisième ensemble d'hyperparamètres avec le spectre mesuré et les N spectres de référence normalisés associés aux substances candidates de mélange, dans lequel le troisième ensemble d'hyperparamètres étant configuré de telle sorte que l'effet de filtrage est atténué par rapport au deuxième ensemble d'hyperparamètres moyennant quoi

les substances sont éliminées jusqu'à ce que toutes les P substances restantes

aient un poids relatif supérieur à un seuil d'importance prédéfini, le poids relatif étant le poids normalisé du résultat du problème d'optimisation ;

r) calculant une pluralité de lots de composition à partir des spectres de référence normalisés associés aux P substances restantes de telle sorte qu'un lot de composition est généré pour chaque sous-ensemble possible des P substances restantes qui comporte la substance restante avec le poids relatif le plus élevé ;

s) résolvant le problème d'optimisation pour chaque lot de composition et le spectre mesuré avec le premier ensemble d'hyperparamètres résultant en un spectre composite par lot de composition ; et

t) évaluant la qualité de chaque spectre composite en évaluant les résultats du problème d'optimisation, tels qu'obtenus à l'aide du premier ensemble d'hyperparamètres, avec ladite métrique de similarité évaluant la correspondance spectrale du spectre composite respectif avec le spectre mesuré, et fournissant des substances associées aux spectres composites ayant une qualité supérieure à un seuil de qualité prédéfini en guise d'ensemble de substances (R2) contenues dans l'échantillon.

11. Système selon la revendication 10, comprenant en outre :

un module de recherche multi-substances étendue (140) configuré pour effectuer une recherche multi-substances étendue en :

u) composant une pluralité de lots de filtre dans lequel chaque lot de filtre comporte un sous-ensemble P de premier rang du nombre prédéfini M de spectres de référence à partir de la liste HIT (290) de l'étape o), les spectres de référence associés à la une ou plusieurs substances fournies par l'étape t), et un nombre prédéfini des spectres de référence restants à partir de la bibliothèque (300), et normalisant les spectres de référence de chaque lot de filtre ;

v) résolvant le problème d'optimisation avec le deuxième ensemble d'hyperparamètres pour chaque lot de filtre (Lot_l à Lot_N) et le spectre mesuré (211) ;

w) consolidant les ensembles de résultats de chaque lot de filtre en éliminant les substances redondantes du résultat consolidé dans un ensemble consolidé de substances candidates de mélange ; et

x) réappliquant les étapes p) à t) à l'aide des spectres de référence associés à l'ensemble consolidé de substances candidates de mélange au lieu des spectres de référence sélectionnés de l'étape p).

12. Système selon l'une quelconque de la revendication 10 ou 11, dans lequel la spectroscopie optique est choisie parmi l'une quelconque de : spectroscopie RAMAN, spectroscopie infrarouge, et spectroscopie laser à cascade quantique

13. Système selon l'une quelconque des revendications 10 à 12, dans lequel la métrique de similarité est une moyenne pondérée d'une métrique de similarité d'ajustement et d'une métrique de similarité de pic.

FIG. 1

substance
filtering
2100

substance reduction
and importance
weighting 2200

2000A

result
calculation
2300

FIG. 2A

FIG. 2C

HIT List Top M Entries

Substance
Filter

2310'

Top K

300

211

311

2000B

pre-processing 2100'

SSS 2200'

HIT list 290

Top M

substance filtering
2310'

substance reduction
and importance
weighting 2320'

result
calculation
2330'

LMSS 2300'

batchwise
substance filtering
2410'

substance reduction
and importance
weighting 2420'

result
calculation
2430'

EMSS 2400'

FIG. 2B

EMSS 2400'

**Filter Batch N** | All Batch Subst. | Remaining Library Spectra ZXX - ZYY → Filter → Top K — RFBN

**Filter Batch 2** | All Batch Subst. | Remaining Library Spectra ZAA – ZBB → Filter → Top K — RFB2

**Filter Batch 1** | All Batch Subst. | Remaining Library Spectra Z1 – ZAA → Filter → Top K

consolidated substances — CMSC

291 — **HIT List Top M** | Z3 | Z424 | Z878 | Z444 | ....

311r — **Remaining Library Spectra** | Z1 | Z2 | Z4 | Z5 | ....

All Batch Substances HIT List Top P + Fast Multi-Substance Search Results

FIG. 3

1000

obtaining measured spectrum, and accessing library with reference spectra of known pure substances — 1100

pre-processing the measured spectrum and the reference spectra — 1200

single substance search

> solving optimization problem with a first set of hyperparameters for possible pairs of spectra 1310
>
> determining similarity score for matching result and ranking reference spectra 1320

— 1300

limited multi-substance search

> selecting predefined number of reference spectra from ranking result and standardizing selected reference spectra 1410
>
> solving optimization problem with a second set of hyperparameters emphasizing substance filtering to result in a set of mixture substance candidates 1420
>
> solving optimization problem with a third set of hyperparameters with the measured spectrum and standardized reference spectra associated with mixture substance candidates with attenuated filtering effect to discard substances with a relative weight below a predefined threshold 1430

— 1400

*Continued on FIG. 4B*

# FIG. 4A

<u>1000</u>

Continued from FIG. 4A

computing composition batches for each combination of normalized reference spectra associated with the respective remaining substances which include the substance with the highest relative weight <u>1440</u>

1400

for each composition batch solving the optimization problem with the first set of hyperparameters where filtering is eliminated, resulting in a composite spectrum per composition batch <u>1450</u>

evaluating the quality of each composite spectrum and providing one or more substances associated with composite spectra having a quality above a predefined quality threshold as substances included in the sample <u>1460</u>

extended multi-substance search

composing filter batches wherein each filter batch includes reference spectra associated with a top-ranked subset of the set of mixture substance candidates and a predefined number of the remaining reference spectra and standardizing reference spectra of each filter batch <u>1510</u>

1500

solving the optimization problem with second set of fixed hyperparameters for each filter batch such that substance filtering is emphasized to result in a set of mixture substance candidates <u>1520</u>

consolidating result sets of each filter batch into a consolidated set of mixture substance candidates <u>1530</u>

reapplying steps 1420 to 1460 by using the reference spectra associated with the consolidated set of mixture substance candidates instead of the selected reference spectra of step 1420 <u>1540</u>

# FIG. 4B

FIG. 5A

FIG. 5B

FIG. 5C

FIG. 6

FIG. 7A

FIG. 7B

FIG. 7C

FIG. 8A

FIG. 8B

FIG. 8C

FIG. 9

EP 4 467 944 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016259792 A1

**Non-patent literature cited in the description**

- **K. NIPP** ; **D. STOFFER**. Lineare Algebra: Eine Einführung für Ingenieure unter besonderer Berück-sichtigung numerischer Aspekte. Hochschulverlag AG, 2002